# EUROPEAN PATENT SPECIFICATION

(11) **EP 1 936 376 B1**
(45) Date of publication and mention of the grant of the patent: **11.04.2012**
(21) Application number: 08005572.6
(22) Date of filing: 06.02.2004
(51) Int. Cl.: G01N 33/53, A61P 15/06, A61K 31/20, A61K 31/57, A61K 31/202, G01N 33/68

(54) **Screening and treatment methods for prevention of preterm delivery**
Screening und Behandlungsverfahren zur Vorbeugung von Frühgeburten
Procédés de criblage et de traitement pour la prévention des accouchements prématurés

(30) Priority: 06.02.2003 US 446288 P; 28.04.2003 US 466526 P; 28.07.2003 US 490706 P
(43) Date of publication of application: 25.06.2008
(62) Divisional of application: 04709003.0
(73) Proprietor: Hologic Inc., Marlborough, MA 01752 (US)
(72) Inventor: Hickok, Durlin, Seattle, WA 98112-6427 (US); Hussa, Robert, Sunnyvale California 94089 (US); Fischer-Colbrie, Mark, Cupertino California 95014 (US); Anderson, Emory V., Denville California 94506 (US); Senyei, Andrew E., La Jolia California 92037 (US)
(74) Representative: Spencer, Matthew Peter

(56) References cited:
- WO-A-01/01899
- WO-A-95/12406
- YEMINI M ET AL: "Prevention of premature labor by 17 alpha-hydroxyprogesterone caproate." AMERICAN JOURNAL OF OBSTETRICS AND GYNECOLOGY 1 MAR 1985, vol. 151, no. 5, 1 March 1985 (1985-03-01), pages 574-577, XP008100826 ISSN: 0002-9378
- KEIRSE M J N C: "PROGESTOGEN ADMINISTRATION IN PREGNANCY MAY PREVENT PRETERM DELIVERY" 19900101; 19900000, vol. 97, no. 2, 1 January 1990 (1990-01-01), pages 149-154, XP009042565
- OLSEN S F ET AL: "Randomised clinical trials of fish oil supplementation in high risk pregnancies. Fish Oil Trials In Pregnancy (FOTIP) Team." BJOG : AN INTERNATIONAL JOURNAL OF OBSTETRICS AND GYNAECOLOGY MAR 2000, vol. 107, no. 3, March 2000 (2000-03), pages 382-395, XP002511482 ISSN: 1470-0328
- ASCARELLI M H ET AL: "Use of fetal fibronectin in clinical practice" OBSTETRICAL AND GYNECOLOGICAL SURVEY, WILLIAMS & WILKINS, BALTIMORE, MD, US, vol. 52, no. 4 Suppl, 1 April 1997 (1997-04-01), pages S1-S11, XP008099888 ISSN: 0029-7828
- ANDERSEN H F: "Use of fetal fibronectin in women at risk for preterm delivery" CLINICAL OBSTETRICS AND GYNECOLOGY, HARPER AND ROW, HAGERSTOWN, MD, US, vol. 43, no. 4, 1 December 2000 (2000-12-01), pages 746-758, XP008099875 ISSN: 0009-9201
- HONEST HONEST ET AL: "Accuracy of cervicovaginal fetal fibronectin test in predicting risk of spontaneous preterm birth: Systematic review", BMJ, vol. 325, no. 7359, 10 August 2002 (2002-08-10), pages 301-304, XP007913823, ISSN: 0959-8138
- MEIS P J ET AL: "Prevention of recurrent preterm delivery by 17 alpha-hydroxyprogesterone caproate", NEW ENGLAND JOURNAL OF MEDICINE 20030612 US LNKD- DOI:10.1056/NEJMOA035140, vol. 348, no. 24, 12 June 2003 (2003-06-12), pages 2379-2385, XP002630475, ISSN: 0028-4793
- O'BRIEN J M ET AL: "Progesterone vaginal gel for the reduction of recurrent preterm birth: Primary results from a randomized, double-blind, placebo-controlled trial", ULTRASOUND IN OBSTETRICS AND GYNECOLOGY 200710 GB LNKD- DOI:10.1002/UOG.5158, vol. 30, no. 5, October 2007 (2007-10), pages 687-696, XP007918096, ISSN: 0960-7692

## Description

### FIELD OF THE INVENTION

Progestational agents for the prevention of preterm delivery are provided in combination with methods for screening women to identify subjects having an increased risk for preterm delivery, which includes impending and imminent delivery.

### BACKGROUND

Preterm neonates account for a majority of the morbidity and mortality in newborns without congenital anomalies. Accordingly, determination of subjects at risk for impending preterm births and appropriate treatment is critical for increasing neonatal survival of preterm infants. Due to the subtlety of symptoms associated with preterm delivery, many subjects are not diagnosed as having an increased risk of preterm delivery until later in their pregnancies.

A number of pharmaceutical compounds that can delay delivery have been introduced. Among these are progestational agents. There is evidence that the effectiveness of the progestational agents is improved when the agents are taken early on. The use of such agents, however, is not without risk. For example, progestational agents have been linked to a possible increase in the risk of malformation (e.g., cardiac, neurological, neural tube and others) and esophageal atresia.

Since there are benefits and risks to administration of these agents, there is a need to identify pregnant females who are candidates for such treatment.

Yemini M. *et al* (1985) *Am J Obstet Gynecol; 151(5): 574-7* and Keirse M. (1990) *Br J Obstet Gynecol; 97: 149-54* disclose the use of 17-α-hydroxyprogesterone caproate to prevent preterm delivery.

Honest H. *et al* (2002) *BMJ; 325: 301-10* and Ascarelli MH and Morrison JC (1997) *Obstet Gynecol Surv; 52(4 Suppl):S1-12* disclose the use fetal fibronectin in clinical practice to predict the risk of spontaneous preterm birth.

### SUMMARY

The present invention relates to 17-α-hydroxyprogesterone or 17-α-hydroxyprogesterone caproate for use in delaying delivery in a subject at risk of preterm delivery as defined in the appended claims.

Provided herein are methods, combinations and kits for identifying pregnant females, particularly early in pregnancy, who are candidates for treatment with progestational agents. As provided herein such candidates are those who exhibit an increased risk for preterm delivery as evidenced by markers of preterm delivery such as fetal fibronectin (fFN). Samples can be tested early in the pregnancy, generally as early as 20 weeks or later of gestation, including up to 36 weeks, after which delivery is not considered preterm.

In particular, provided herein are methods of screening a subject where a body fluid sample, such as a sample of urine, blood, plasma, saliva, cervical fluid or vaginal fluid, is obtained from the subject and the level of fetal fibronectin therein is detected. In one embodiment, the subject is a subject at risk for preterm delivery. If the level of fetal fibronectin meets a predetermined selection criterion indicative of such risk of preterm delivery, a therapeutically effective amount of a progestational agent is administered to the subject. In certain embodiments, the sample is obtained after 20 weeks gestation. The progestational agent contains at least one progesterone or derivative thereof, such as 17-α-hydroxyprogesterone caproate or 17-α-hydroxyprogesterone. In a further embodiment, the therapeutically effective amount of the progestational agent is administered at of a dosage of at least about 100 mg/week, 250 mg/week, 500 mg/week, 1000 mg/week, 1500 mg/week, or 2000 mg/week of the progestational agent. Alternatively, the therapeutically effective amount of the progestational agent is administered at a dosage of at least about 10 mg/day, 25 mg/day, 80 mg/day, 100 mg/day, 200 mg/day, or 300 mg/day, or more, of the progestational agent. The progestational agent can be administered by any suitable route, including orally, by intramuscular injection, transdermally, or intranasally. The progestational agent is administered after 20 weeks, after about 28 weeks, or after about 35 weeks gestation. The administration of the progestational agent is stopped at 36 weeks gestation or at the onset of spontaneous labor. In another embodiment, the predetermined selection criterion includes a threshold value, where the progestational agent is administered to the subject when the level of fetal fibronectin is above the threshold value (e.g., about 50 ng/mL). In still other embodiments, the level of fetal fibronectin is detected using an immunoassay, such as, but are not limited to, a homogeneous or heterogeneous, sandwich or competitive assay.

Further provided herein are combinations for screening a subject containing an anti-(fetal restricted antigen) antibody; an anti-(fetal restricted antigen class) antibody; and a progestational agent. In one embodiment, either the anti-(fetal restricted antigen) antibody or the anti-(fetal restricted antigen class) antibody is adhered to an insoluble support. The anti-(fetal restricted antigen) antibody is an anti-(fetal fibronectin) antibody and the anti-(fetal restricted antigen class) antibody is an anti-fibronectin antibody.

Additionally provided herein are kits for screening a subject containing an anti-(fetal restricted antigen) antibody; an anti-(fetal restricted antigen class) antibody; a progestational agent; and a device for obtaining a sample from the subject. In one embodiment, either the anti-(fetal restricted antigen) antibody or the anti-(fetal restricted antigen class) antibody is adhered to an insoluble support. The anti-(fetal restricted antigen) antibody contains an anti-(fetal fibronectin) antibody and the anti-(fetal restricted antigen class) antibody contains an anti-fibronectin antibody.

Even further provided herein are methods of screening a subject wherein the level of a fetal restricted antigen is determined in a first body fluid sample, such as urine, blood, plasma, saliva, cervical fluid and vaginal fluid, obtained from the subject. The level of an insulin-like growth factor binding protein one also is determined in a second body fluid sample, such as urine, blood, plasma, saliva, cervical fluid and vaginal fluid, from the subject. A therapeutically effective amount of a progestational agent is administered to the subject when the level of fetal restricted antigen meets a first predetermined selection criterion and the level of insulin-like growth factor binding protein one meets a second predetermined selection criterion. The first and second samples are optionally the same. The fetal restricted antigen is fetal fibronectin.

Also provided herein are combinations for screening a subject containing an anti-(fetal restricted antigen) antibody; an anti-(insulin-like growth factor binding protein one) antibody and/or reagents for detecting or measuring estriol, particularly unconjugated estriol; and a progestational agent. The anti-(fetal restricted antigen) antibody contains an anti-(fetal fibronectin) antibody.

Further provided herein are kits for screening a subject containing an anti-(fetal restricted antigen) antibody; an anti-(insulin-like growth factor binding protein one) antibody; a progestational agent; and a device for obtaining a sample from the subject. The anti-(fetal restricted antigen) antibody contains an anti-(fetal fibronectin) antibody.

Even further provided herein are methods of screening a subject where the level of a fetal restricted antigen is determined in a first body fluid sample, such as urine, blood, plasma, saliva, cervical fluid and vaginal fluid, obtained from the subject. The level of estriol also is determined in a second body fluid sample, such as urine, blood, plasma, saliva, cervical fluid and vaginal fluid, obtained from the subject. A therapeutically effective amount of a progestational agent is to be administered to the subject when the level of fetal restricted antigen meets a first predetermined selection criterion and the level of estriol meets a second predetermined selection criterion. The first and second samples are optionally the same. The fetal restricted antigen is fetal fibronectin.

Also provided herein are combinations and kits for screening a subject containing an anti-(preterm delivery marker) antibody and a progestational agent. In various embodiments, the antibody is an anti-(fetal restricted antigen) antibody such as anti-(fetal fibronectin) antibody, or an anti-(fetal restricted antigen class) antibody such as an anti-fibronectin antibody. The combinations and kits can contain two or more antibodies, including an anti-(fetal restricted antigen) antibody such as anti-(fetal fibronectin) antibody, an anti-(fetal restricted antigen class) antibody such as an anti-fibronectin antibody, and optionally an anti-estriol antibody, or an anti-(membrane rupture) antibody such as an anti-(insulin-like growth factor binding protein one) antibody. The antibodies of the combinations and kits can be conjugated, unconjugated or immobilized to a solid support. Combinations and kits provided herein can also include one or more progesterone-related agents such as 17-α-hydroxyprogesterone or 17-α-hydroxyprogesterone caproate.

Also provided are combinations containing a kit for detecting the presence of a fetal-restricted antigen in a sample, and a progesterone-related agent. Such combinations can contain kits that contain an antibody that specifically binds to a fetal restricted antigen or the kit can contain an immunoassay test strip to detect a fetal restricted antigen in a sample. The combination can contain a sample collection device, where the sample contains a body fluid or a swab of the posterior fornix, the cervical canal, the ectocervix and/or the external cervical os. A progesterone-related agent of the combination is 17-α-hydroxyprogesterone or 17-α-hydroxyprogesterone caproate.

Also provided herein is a use of the combinations or kits provided herein in a test for detecting the level of a fetal-restricted hormone and a progesterone-related agent for delaying preterm delivery.

Further provided herein is a medicament for treating a subject, comprising a progestational agent, wherein the subject has an increased risk of preterm delivery, as determined by the level of a fetal restricted antigen in a sample from the subject. Also provided is a medicament for treating a subject, comprising a progestational agent, wherein the progestational agent is administered to a subject having an increased risk of preterm delivery, as determined by the level of a fetal restricted antigen in a sample from the subject.

### DETAILED DESCRIPTION

- A.: Definitions
- B.: Selecting the Subject and Obtaining the Sample
- C.: Determining the risk of preterm or imminent delivery
1. Determining the Level of Fetal Restricted Antigen
a. Assays
b. Test Device
c. Immunoassay Test Strip
d. Test Strip Housing
e. Antibodies
f. Conjugation of the Antibody to a Label
g. Measurement of Fetal Fibronectin
h. Test Strip for Measuring fFN and Cellular Fibronectin
i. Antibodies for Fetal Fibronectin
j. Fetal Fibronectin Assay Procedure
k. Determining the Selection Criterion
2. Determining the Level of Estriol
3. Determining the Level of Insulin-Like Grow actor Binding Protein One
- D.: Administering the Progestational Agent
- E.: Combinations and Kits
- F.: Examples

### A. Definitions

Unless defined otherwise, all technical and scientific terms used herein have the same meaning as is commonly understood by one of skill in the art to which the inventions belong. In the event that there are a plurality of definitions for terms herein, those in this section prevail. Where reference is made to a URL or other such identifier or address, it understood that such identifiers can change and particular information on the internet can come and go, but equivalent information is known and can be readily accessed, such as by searching the internet and/or appropriate databases. Reference thereto evidences the availability and public dissemination of such information.

As used herein, a subject or subject refers to pregnant women for whom diagnosis and/or treatment is contemplated.

As used herein, risk refers to a predictive process in which the probability of a particular outcome is assessed.

As used herein, the term "preterm delivery" refers to delivery that occurs from about 20 weeks gestation to about 37 weeks gestation. The number of weeks gestation (i.e., gestational age) can be determined using any of a number of conventional methods. For example, the gestational age can be calculated from the first day of the last menstruation.

As used herein, risk of imminent delivery refers to the risk of delivering within a predetermined time frame, such as within 7, 14, 21, 28 days. Generally, the risk of imminent delivery is assessed to identify a risk for preterm delivery.

As used herein, the term "preterm delivery marker" refers to a compound that, when present at or beyond a threshold amount, indicates an increased risk of preterm or imminent delivery. Exemplary preterm delivery markers include a fetal restricted antigen such as fetal fibronectin, and estriol. Threshold amounts for a preterm delivery marker can be a minimum threshold amount, where a level of preterm delivery marker that is equal to or crosses the minimum threshold amount is a level of preterm delivery marker that is equal to or above the threshold amount. Threshold amounts for a preterm delivery marker can be a maximum threshold amount, where a level of preterm delivery marker that is equal to or crosses the maximum threshold amount is a level of preterm delivery marker that is equal to or below the threshold amount.

As used herein, the term "fetal restricted antigen" refers to a uniquely fetal or placental derived material, which is either not present in maternal serum, plasma or urine, or is not present in significant amounts in maternal serum, plasma or urine. Typically it is an antigen that is produced by a fetus or placenta that is not produced in significant amounts or not produced by the mother. An example of a fetal restricted antigen is fetal fibronectin. Fetal fibronectin preferentially binds, for example, to antibodies specific therefor. Exemplary of such antibodies is FDC-6 monoclonal antibody (see, e.g., Matsuura (1985) Proc. Natl. Acad. Sci. U.S.A. 82:6517-6521). Production of the hybridoma (deposited at the American Type Culture Collection as accession number ATCG HB 9018), which produces FDC-6 antibody is described in detail in U.S. Patent No. 4,894,326, issued January 16, 1990, to Matsuura *et al.*

As used herein, the term "membrane rupture marker" refers to a compound that, when present at or beyond a threshold amount, indicates membrane rupture in a pregnant subject. An exemplary preterm delivery marker is insulin-like growth factor binding protein one.

As used herein, the term "agent" broadly includes any substance that is capable of producing an effect.

As used herein, "hormone" and "steroid" are used interchangeably and refer to a group of chemical messengers that are synthesized by specific tissues and alter the activity of target organs or cells.

As used herein, the term "fetal restricted antigen class" refers to a class or group of antigens of which the fetal restricted antigen is a member. For example, fetal fibronectin is a fetal restricted member of the fibronectin group or class.

As used herein, fetal fibronectin is a fetal restricted antigen found in placenta, amniotic fluid and fetal connective tissue. It differs structurally from adult fibronectins. Fetal fibronectin is not present in significant quantities in maternal plasma or serum. Fetal fibronectin can be captured with a general binding antibody, such as an anti-fibronectin antibody, or an anti-fetal restricted antigen antibody, such as anti-fetal fibronectin antibody.

As used herein, the term "IGFBP-1 " refers to insulin-like growth factor binding protein one (also known as pregnancy-associated endometrial α-globulin (αPEG), and placental protein-12 (PP-12) (Waites et al, J. Clinical Endocrinology and Metab. 67:1100 1986). Assays that detect IGFBP-1 are intended to detect the level of endogenous (native) IGFBP- present in the maternal blood. Exogenous IGFBP-1 (IGFBP-1 from a source extrinsic to the blood sample) can be added to various assays to provide a label or to compete with the native IGFBP-1 in binding to an anti-IGFBP-1 antibody. One of skill in the art will appreciate that an IGFBP-1 mimetic can be used in place of exogenous IGFBP-1. An "IGFBP-1 mimetic," as used herein, refers to a molecule that bears one or more IGFBP-1 epitopes such that it is specifically bound by an antibody that specifically binds native IGFBP-1.

As used herein, estriol is usually the predominant estrogenic metabolite found in urine and can be related to fetal distress. Accordingly, the detection of estriol serum levels in pregnant women provides information on fetal status during pregnancy. The clinical significance of estrogenic hormones is discussed by K. S. McCarty et al. in Regulatory Mechanisms in Breast Cancer, Chapter 9, Kluwer Academic Publishers, Boston, 1991.

As used herein, the term "progestational agent" refers 17-α-hydroxyprogesterone or 17-α-hydroxyprogesterone caproate.

As used herein, a derivative of a compound includes a salt, ester, enol ether, enol ester, solvate or hydrate thereof that can be readily prepared by those of skill in this art using known methods for such derivatization. Salts include, but are not limited to, amine salts, such as but not limited to N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethylbenzimidazole, diethylamine and other alkylamines, piperazine and tris(hydroxymethyl)aminomethane; alkali metal salts, such as but not limited to lithium, potassium and sodium; alkali earth metal salts, such as but not limited to barium, calcium and magnesium; transition metal salts, such as but not limited to zinc; and other metal salts, such as but not limited to sodium hydrogen phosphate and disodium phosphate; and also including, but not limited to, salts of mineral acids, such as but not limited to hydrochlorides and sulfates; and salts of organic acids, such as but not limited to acetates, lactates, malates, tartrates, citrates, ascorbates, succinates, butyrates, valerates and fumarates. Esters include, but are not limited to, alkyl, alkenyl, alkynyl, aryl, heteroaryl, aralkyl, heteroaralkyl, cycloalkyl and heterocyclyl esters of acidic groups, including, but not limited to, carboxylic acids, phosphoric acids, phosphinic acids, sulfonic acids, sulfinic acids and boronic acids. Enol ethers include, but are not limited to, derivatives of formula C = C(OR) where R is hydrogen, alkyl, alkenyl, alkynyl, aryl, heteroaryl, aralkyl, heteroaralkyl, cycloalkyl and heterocyclyl. Enol esters include, but are not limited to, derivatives of formula C = C(OC(O)R) where R is hydrogen, alkyl, alkenyl, alkynyl, aryl, heteroaryl, aralkyl, heteroaralkyl, cycloalkyl and heterocyclyl. Solvates and hydrates are complexes of a compound with one or more solvent or water molecule, preferably 1 to about 100, more preferably 1 to about 10, most preferably one to about 2, 3 or 4, solvent or water molecules.

As used herein, an immunoassay is referred to as any method using a preferential binding of an antigen with a second material (i.e., a binding partner, usually an antibody, antibody fragment or another substance having an antigen binding site), which binds preferentially with an epitope of the antigen. Preferential binding, as used herein, refers to binding between binding partners that is selective and generally specific, and that demonstrates less than about 10%, generally less than about 5%, cross-reactive nonspecific binding. For example, when the analyte is fetal fibronectin, the anti-(fetal fibronectin) antibody is less than 10%, and generally less than 5%, cross-reactive with adult fibronectins. The immunoassay methods provided herein include any known to those of skill in the art, including, but not limited to, sandwich, competition, agglutination or precipitation.

As used herein, the term "bind" or "binding" is used to refer to the binding between two compounds, such as the binding of an Ad5 shaft motif with HSP (Heparin Sulfate Proteoglycans), with a K_{d} in the range of 10⁻² to 10⁻¹⁵ mole/l, generally, 10⁻⁶ to 10⁻⁵, 10⁻⁷ to 10⁻¹⁵ and typically 10⁻⁸ to 10⁻¹⁵ (and/or a Kₐ of 10⁵-10¹², 10⁷-10¹², 10⁸-10¹² I/mole).

As used herein, specific binding or selective binding means that the binding of two compounds (kₐ or K_{eq}) is at least 2-fold, generally, 5, 10, 50, 100 or more-fold, greater than for another receptor. A statement that a particular viral vector is targeted to a cell or tissue means that its affinity for such cell or tissue in a host or *in vitro* is at least about 2-fold, generally, 5, 10, 50, 100 or more-fold, greater than for other cells and tissues in the host or under the *in vitro* conditions.

As used herein, the term "preferentially binding" includes antibodies and fragments thereof that have less than 10% and generally less than 5 % cross-reactivity.

As used herein, a solid support refers to the material to which an antibody is linked. A variety of materials can be used as the solid support. The support materials include any material that can act as a support for attachment of the molecules of interest. Such materials are known to those of skill in this art. These materials include, but are not limited to, organic or inorganic polymers, natural and synthetic polymers, including, but not limited to, agarose, cellulose, nitrocellulose, cellulose acetate, other cellulose derivatives, dextran, dextran-derivatives and dextran co-polymers, other polysaccharides, glass, silica gels, gelatin, polyvinyl pyrrolidone, rayon, nylon, polyethylene, polypropylene, polybutlyene, polycarbonate, polyesters, polyamides, vinyl polymers, polyvinylalcohols, polystyrene and polystyrene copolymers, polystyrene cross-linked with divinylbenzene or the like, acrylic resins, acrylates and acrylic acids, acrylamides, polyacrylamides, polyacrylamide blends, co-polymers of vinyl and acrylamide, methacrylates, methacrylate derivatives and co-polymers, other polymers and co-polymers with various functional groups, latex, butyl rubber and other synthetic rubbers, silicon, glass, paper, natural sponges, insoluble protein, surfactants, red blood cells, metals, metalloids, magnetic materials, or other commercially available media.

As used herein, antibody refers to an immunoglobulin, whether natural or partially or wholly synthetically produced, including any derivative thereof that retains the specific binding ability of the antibody. Hence antibody includes any protein having a binding domain that is homologous or substantially homologous to an immunoglobulin binding domain. For purposes herein, antibody includes antibody fragments, such as Fab fragments, which are composed of a light chain and the variable region of a heavy chain. Antibodies include members of any immunoglobulin class, including IgG, IgM, IgA, IgD and IgE. Also contemplated herein are receptors that specifically bind to a sequence of amino acids.

Hence for purposes herein, any set of pairs of binding members, referred to generically herein as a capture agent/polypeptide tag, can be used instead of antibodies and epitopes per se. The methods herein rely on the capture agent/tag, such as and antibody/polypeptide tag, for their specific interactions, any such combination of receptors/ligands (tag) can be used. Furthermore, for purposes herein, the capture agents, such as antibodies employed, can be binding portions thereof.

As used herein, a monoclonal antibody refers to an antibody secreted by a hybridoma clone. Because each such clone is derived from a single B cell, all of the antibody molecules are identical. Monoclonal antibodies can be prepared using standard methods known to those with skill in the art (see, *e.g.,* Kohler et al. Nature 256:495 (1975) and Kohler et al. Eur. J. Immunol. 6:51 1 (1976)). For example, an animal is immunized by standard methods to produce antibody-secreting somatic cells. These cells are then removed from the immunized animal for fusion to myeloma cells.

Somatic cells with the potential to produce antibodies, particularly B cells, are suitable for fusion with a myeloma cell line. These somatic cells can be derived from the lymph nodes, spleens and peripheral blood of primed animals. Specialized myeloma cell lines have been developed from lymphocytic tumors for use in hybridoma-producing fusion procedures (Kohler and Milstein, Eur. J. Immunol. 6:511 (1976); Shulman et al. Nature 276: 269 (1978); Volk et al. J. Virol. 42: 220 (1982)). These cell lines have been developed for at least three reasons. The first is to facilitate the selection of fused hybridomas from unfused and similarly indefinitely self-propagating myeloma cells. Usually, this is accomplished by using myelomas with enzyme deficiencies that render them incapable of growing in certain selective media that support the growth of hybridomas. The second reason arises from the inherent ability of lymphocytic tumor cells to produce their own antibodies. The purpose of using monoclonal techniques is to obtain fused hybrid cell lines with unlimited life spans that produce the desired single antibody under the genetic control of the somatic cell component of the hybridoma. To eliminate the production of tumor cell antibodies by the hybridomas, myeloma cell lines incapable of producing endogenous light or heavy immunoglobulin chains are used. A third reason for selection of these cell lines is for their suitability and efficiency for fusion. Other methods for producing hybridomas and monoclonal antibodies are well known to those of skill in the art.

As used herein, antibody fragment refers to any derivative of an antibody that is less than full length, retaining at least a portion of the full-length antibody's specific binding ability. Examples of antibody fragments include, but are not limited to, Fab, Fab', F(ab)₂, single-chain Fvs (scFv), Fv, dsFv diabody and Fd fragments. The fragment can include multiple chains linked together, such as by disulfide bridges. An antibody fragment generally contains at least about 50 amino acids and typically at least 200 amino acids.

As used herein, a Fv antibody fragment is composed of one variable heavy domain (V_{H}) and one variable light (V_{L}) domain linked by noncovalent interactions.

As used herein, a dsFv refers to a Fv with an engineered intermolecular disulfide bond, which stabilizes the V_{H}-V_{L} pair.

As used herein, an F(ab)₂ fragment is an antibody fragment that results from digestion of an immunoglobulin with pepsin at pH 4.0-4.5; it can be recombinantly produced.

As used herein, a Fab fragment is an antibody fragment that results from digestion of an immunoglobulin with papain; it can be recombinantly produced.

As used herein, scFvs refer to antibody fragments that contain a variable light chain (V_{L}) and variable heavy chain (V_{H}) covalently connected by a polypeptide linker in any order. The linker is of a length such that the two variable domains are bridged without substantial interference. Exemplary linkers are (Gly-Ser)ₙ residues with some Glu or Lys residues dispersed throughout to increase solubility.

As used herein, hsFv refers to antibody fragments in which the constant domains normally present in an Fab fragment have been substituted with a heterodimeric coiled-coil domain (see, *e.g.,* Arndt et al. (2001) J Mol Biol. 7:312:221-228).

As used herein, diabodies are dimeric scFv; diabodies typically have shorter peptide linkers than scFvs, and they preferentially dimerize.

As used herein, humanized antibodies refer to antibodies that are modified to include "human" sequences of amino acids so that administration to a human does not provoke an immune response. Methods for preparation of such antibodies are known. For example, the hybridoma that expresses the monoclonal antibody is altered by recombinant DNA techniques to express an antibody in which the amino acid composition of the non-variable regions is based on human antibodies. Computer programs have been designed to identify such regions.

As used herein, a mobilizable antibody or fragment thereof refers to an antibody present on solid support such as a test strip, which, upon contact with a liquid, such as material from a sample, the antibody is not immobilized onto the solid support (e.g., the antibody is dissolved into the sample).

As used herein, a composition refers to any mixture. It can be a solution, a suspension, liquid, powder, a paste, aqueous, non-aqueous or any combination thereof.

As used herein, a therapeutically effective amount of a compound for treating a particular disease is an amount that is sufficient to ameliorate, or in some manner reduce the symptoms associated with the disease or condition. Such amount can be administered as a single dosage or can be administered according to a regimen, whereby it is effective. The amount can cure the disease or condition but, typically, is administered in order to ameliorate the symptoms of the disease or condition. Repeated administration can be required to achieve the desired amelioration of symptoms.

As used herein, a "sample" generally refers to anything which can contain an analyte for which an analyte assay is desired. The sample can be a biological sample, such as a biological or body fluid or a biological tissue. Examples of body fluids include urine, blood, plasma, serum, saliva, cervical fluid, vaginal fluid, semen, stool, sputum, cerebral spinal fluid, tears, mucus, amniotic fluid, swabs of cells or of a body fluid, and others. Biological tissues are aggregate of cells, usually of a particular kind together with their intercellular substance that form one of the structural materials of a human, animal, plant, bacterial, fungal or viral structure, including connective, epithelium, muscle and nerve tissues. Examples of biological tissues also include organs, tumors, lymph nodes, arteries and individual cell(s).

As used herein, a combination refers to any association between two or among more items that are used for a common or related purpose. For example, a combination can include a test for preterm delivery and a progestational agent, where the common purpose is to delay delivery.

As used herein, kits refer two or more items in a package or set, optionally including instructions and/or reagents for their use.

For clarity of disclosure, and not by way of limitation, the detailed description is divided into the subsections that follow.

### B. Selecting the Subject and Obtaining the Sample

The present methods can be used on any pregnant woman following 20 weeks gestation. In addition to screening any woman to determine whether she is at risk of preterm delivery, the subjects who should be screened are those subjects with clinically intact membranes in a high risk category for preterm delivery, and especially, those women whose pregnancies are not sufficiently advanced to ensure delivery of a healthy fetus.

In addition, there are a large number of factors known to be associated with the risk of preterm delivery. Those factors include, but are not limited to, multiple fetus gestations; incomplete cervix; uterine anomalies; polyhydramnios; nulliparity; previous preterm rupture of membranes or preterm labor; preeclampsia; first trimester vaginal bleeding; little or no antenatal care; and symptoms such as abdominal pain, low backache, passage of cervical mucus and contractions. Any pregnant woman at 20 or more weeks gestation with clinically intact membranes and having one or more risk factors for preterm delivery should be tested throughout the risk period; i.e., until about 37 weeks gestation. Risk factors for spontaneous abortion include gross fetal anomalies, abnormal placental formation, uterine anomalies and maternal infectious disease, endocrine disorder cardiovascular renal hypertension, autoimmune and other immunologic disease, and malnutrition.

A test sample which is to be assayed is removed in the vicinity of the posterior fornix, the cervical canal, the ectocervix and/or the external cervical os. The sample generally comprises fluid and particulate solids, and can contain vaginal or cervical mucus, other vaginal or cervical secretions, cells or cell debris, amniotic fluid, or other fetal or maternal materials. The sample can be removed using any of a variety of techniques including, but not limited to, use of a swab having a dacron or other fibrous tip, aspirator, suction device, lavage device or the like.

Following collection, the sample is transferred to a suitable container for storage and transport to a testing laboratory. The test sample is optionally dispersed in a liquid which preserves the protein analytes which are unstable in the sampled composition. The storage and transfer medium should minimize decline in the protein analyte level during storage and transport. A suitable preserving solution for storage and transfer consists of 0.05 M Tris-HCl, pH 7.4; 0.15 M NaCl, 0.02% NaN₃, 1% BSA, 500 Kallikrein Units/mL of aprotinin, 1 mM phenylmethylsulfonyl fluoride (PMSF) and 5 mM EDTA. Suitable preserving solutions for storage and transfer are known in the art, as exemplified in U.S. Patent No. 4,919,889, issued April 24, 1990. The solution can be used, for example, when detecting fetal fibronectin. Calculations to account for any additional dilution of the samples collected using liquids can be performed as part of the interpretation of the assay procedure.

Alternatively, home and office use devices for immediate processing of the sample can be used. If used, the sample is placed directly in the device and testing is performed within minutes of sample collection. In such cases, the need to stabilize the analyte is minimized and any solution which facilitates performing the assay and is not detrimental to analyte stability can be used. Diagnostic systems and kits known in the art can be used to determine the level of fetal restricted antigen in accordance with the methods provided herein. Examples of diagnostic systems and kits known in the art are provided in U.S. Patent Nos. 6,394,952 and 6,267,722.

### C. Determining the risk of preterm or imminent delivery

In practicing the methods provided herein, one or more markers indicative or a risk of preterm or imminent delivery are assessed. If a marker is determined to be at level or in amount indicative of such risk, a progestational agent is administered. In one embodiment, the methods for determining the risk of preterm delivery include determining the level of fetal restricted antigen ( fetal fibronectin) in a sample. In another embodiment, the methods for determining the risk of preterm delivery include determining the level of fetal restricted antigen ( fetal fibronectin) in a sample and determining the level of estriol in the same sample or a different sample.

### 1. Determining the Level of Fetal Restricted Antigen

### a. Assays

Any assay is intended for use in the systems and methods herein. Such assays include, but are not limited to: nucleic acid detection, including using amplification and non-amplification protocols, any assay that relies on colorimetric or spectrometric detection, including fluorometric and luminescent detection, such as creatine, hemoglobin, lipids, ionic assays, and blood chemistry. Any test that produces a signal, or from which a signal can be generated, that can be detected by a detector, such as a photodetector or a gamma counter, is intended for use in the methods provided herein. Any wavelength is intended to be included.

Immunoassays, including competitive and non-competitive immunoassays, are among those contemplated for determination of the presence or amount of analyte in a subject sample, and are exemplified herein. It is understood that immunoassays are provided for exemplification, and that the methods and systems provided herein have broad applicability to subject test data and other test data.

A number of different types of immunoassays are well known using a variety of protocols and labels. Immunoassays can be homogeneous, i.e. performed in a single phase, or heterogeneous, where antigen or antibody is linked to an insoluble solid support upon which the assay is performed. Sandwich or competitive assays can be performed. The reaction steps can be performed simultaneously or sequentially.
Threshold assays can be performed, where a predetermined amount of analyte is removed from the sample using a capture reagent before the assay is performed, and only analyte levels of above the specified concentration are detected. Assay formats include, but are not limited to, for example, assays performed in test tubes, wells or on immunochromatographic test strips, as well as dipstick, lateral flow or migratory format immunoassays.

Any known immunoassay procedure, particularly those that can be adapted for use in combination with lateral flow devices, can be used in the systems and methods provided herein.

### b. Test Device

Any test that produces a signal, or from which a signal can be generated, is intended for use as part of the methods, combinations, and kits provided herein. Further, any test strips that can be, for example, inspected visually or adapted for use in combination with a reader are contemplated for use in the methods, combinations, and kits provided herein. Such test strip devices as are known to those of skill in the art (see, e.g., U.S. Patent Nos. 5,658,801, 5,656,502, 5,591,645, 5,500,375, 5,252,459, 5,132,097 and many other examples) can be used in systems as described herein.

Typically these test devices are intended for use with biological samples, such as saliva, blood, serum, cerebral spinal fluid, and cervicovaginal samples, for example. Other biological samples, such as food samples, which are tested for contamination, such as by bacteria or insects, also are contemplated. Target analytes include, but are not limited to: nucleic acids, proteins, peptides, such as human immunodeficiency virus (HIV) antigens, antigens indicative of bacterial, such as Salmonella and E. coli, yeast or parasitic infections, apolipoprotein(a) and lipoprotein(a), environmental antigens, human chorionic gonadotropin (hCG), E-3-G, interleukins and other cytokines and immunomodulatory proteins, such as IL-6 and interferon, small nuclear ribonuclear particles (snRNP) antigens, fFN and other indicators, such as insulin-like growth factor binding protein one (IGFBP-1), of pregnancy related disorders.

### c. Immunoassay Test Strip

In one embodiment, an immunoassay test strip that includes a membrane system that defines a liquid flow pathway is employed. Many immunoassay test strips and formats for performing the assays are known in the art and are commercially available (see, e.g., U.S. Patent No. 6,267,722).

Lateral flow test immunoassay devices are among those that can be employed in the methods herein. In such devices, a membrane system forms a single fluid flow pathway along the test strip. The membrane system includes components that act as a solid support for immunoreactions. For example, porous or bibulous or absorbent materials can be placed on a strip such that they partially overlap, or a single material can be used, in order to conduct liquid along the strip. The membrane materials can be supported on a backing, such as a plastic backing. In an exemplary embodiment, the test strip includes a glass fiber pad, a nitrocellulose strip and an absorbent cellulose paper strip supported on a plastic backing.

Antibodies that react with the target analyte and/or a detectable label system are immobilized on the solid support. The antibodies can be bound to the test strip by adsorption, ionic binding, van der Waals adsorption, electrostatic binding, or by covalent binding, by using a coupling agent, such as glutaraldehyde. For example, the antibodies can be applied to the conjugate pad and nitrocellulose strip using standard dispensing methods, such as a syringe pump, air brush, ceramic piston pump or drop-on-demand dispenser. In one embodiment, a volumetric ceramic piston pump dispenser is used to stripe antibodies that bind the analyte of interest, including a labeled antibody conjugate, onto a glass fiber conjugate pad and a nitrocellulose strip. In one embodiment, the test strip contains two or more regions containing antibodies. A first region can contain analyte-specific antibody, where, upon contacting the test strip with material from the sample, the analyte-specific antibody is no longer immobilized on the test strip. A second region can contain analyte-specific antibody that remains immobilized on the test strip up contacting the test strip with material from the sample.

The test strip can or cannot be otherwise treated, for example, with sugar to facilitate mobility along the test strip or with water-soluble non-immune animal proteins, such as albumins, including bovine (BSA), other animal proteins, vvater-soluble polyamino acids, or casein to block non-specific binding sites.

A variety of test device formats also can be employed. Such formats include, for, example, vertical flow test immunoassay devices, which contain a solid support for an immunoreaction. Antibodies that react with a target analyte and/or a detectable label system are immobilized on the solid support. The antibodies can be bound to the test strip by adsorption, ionic binding, van der Waals adsorption, electrostatic binding, or by covalent binding, by using a coupling agent, such as glutaraldehyde. For example, the antibodies can be applied to the conjugate pad and nitrocellulose strip using standard dispensing methods, such as a syringe pump, air brush, ceramic piston pump or drop-on-demand dispenser. In one embodiment, a volumetric ceramic piston pump dispenser is used to stripe antibodies that bind the analyte of interest, including a labeled antibody conjugate, onto a glass fiber conjugate pad and a nitrocellulose strip.

In using the vertical flow device, a sample can be prepared by contacting the sample with a second antibody such as an anti-(fetal restricted antigen) antibody (e.g., an anti-(fetal fibronectin) antibody) or an anti-(fetal restricted antigen class) antibody (e.g., an anti-fibronectin antibody) or a conjugate thereof, and after sufficient time for antigen-antibody complex formation, contacting the sample with the solid support. Such second antibodies that form a complex in the strip of the solid support can serve as indicators of the presence of an analyte such as a fetal restricted antigen (e.g., fetal fibronectin) in a sample.

### d. Test Strip Housing

The test strip optionally can be contained within a housing for insertion into a reflectance reader. Assay devices including a test strip and housing assembly are known in the art, as exemplified in U.S. Patent No. 6,267,722.

In an exemplary embodiment, the test strip housing includes a symbology, such as a bar code that can be associated with data related to the assay device, subject data and/or test run. For example, information associated with the device, such as lot number, expiration date, analyte and intensity value, or information related to the test run, such as date, reflectance value or other such information, can be encoded and associated, such as in a database with a bar code imprinted on the device. Any bar code system that provides the appropriate line thickness and spacing can be used. Code 39 and Code 128 are among the known bar code systems.

In a particular embodiment, Code 39 is used. The bar code is made up of 11 alphanumerics, including 2 alphabetic and 9 numeric characters. The first and last characters are asterisks (*), as is standard in the Code 39 system. The lot number is stored as 1 alpha and 4 numeric codes so that product complaints or questions can be traced to a particular lot number. In the exemplified embodiment, the first character represents the month of production, the second is a digit representing the year of production and the last three are an index value indicating the lot number. Thus, the lot number "A8001 " represents the first device in a lot produced in January, 1998. The next two characters ("01") represent the identity of the analyte as 2 numerics (00-99). This permits the use of up to 100 different analytes with the system. The reflectance intensity value (00-99) is stored as the next two numeric characters ("01"). The intensity value sets the reference threshold for which controls and subject samples can be compared. This eliminates the need to run liquid reference samples on a daily basis. Finally, the cassette expiration date is stored as 1 alpha and 1 numeric code to prevent the use of expired devices. In the example given, an expiration code of "A9" represents an expiration date of January, 1999.

### e. Antibodies

Any antibody, including polyclonal or monoclonal antibodies, or any fragment thereof, such as the Fab fragment, that binds the analyte of interest, is contemplated for use herein. Monoclonal and/or polyclonal antibodies can be used. For example, a mouse monoclonal anti-fetal fibronectin antibody can be used in a labeled antibody-conjugate for detecting fetal fibronectin, and a polyclonal goat anti-mouse antibody also can be used to bind fetal fibronectin to form a sandwich complex. An antibody that binds to the labeled antibody conjugate that is not complexed with fetal fibronectin can be immobilized on the test strip and used as a control antibody. For example, when fetal fibronectin is the analyte, a polyclonal goat anti-mouse IgG antibody can be used.

Antibodies used can include anti-(fetal restricted antigen) antibodies and anti-(fetal restricted antigen class) antibodies, where the antibodies can be conjugated to a label, unconjugated, or immobilized on a solid support. Anti-(fetal restricted antigen) antibodies such as anti-(fetal fibronectin) antibodies are typically monoclonal antibodies. Polyclonal anti-(fetal restricted antigen) antibodies such as anti-(fetal fibronectin) antibodies typically bind two or more epitopes uniquely present in the fetal restricted antigen, relative to other antigens in the antigen class. Anti-(fetal restricted antigen class) antibodies such as anti-fibronectin antibodies can be either polyclonal or monoclonal antibodies.

Preferentially binding antibody fragments suitable for use in the methods described herein can be made from the respective monoclonal or polyclonal antibodies by conventional enzyme or chemical fragmentation procedures. Such procedures are well known (see, e.g., Tijssen, P. LABORATORY TECHNIQUES IN BIOCHEMISTRY AND MOLECULAR BIOLOGY: PRACTICE AND THEORIES OF ENZYME IMMUNOASSAYS. New York: Elsevier (1985)).

### f. Conjugation of the Antibody to a Label

An antibody conjugate containing a detectable label can be used to bind the analyte of interest. The detectable label used in the antibody conjugate can be any physical or chemical label capable of being detected on a solid support using a reader, such as a reflectance reader, and capable of being used to distinguish the reagents to be detected from other compounds and materials in the assay.

Suitable antibody labels are well known to those of skill in the art. The labels include, but are not limited to colored labels, such as colored particles and colloidal metals, such as latex beads and colloidal gold; enzyme-substrate combinations that produce color (or fluorescence or other electromagnetic radiation) upon reaction. Colored particles, such as latex particles, colloidal metal or metal or carbon sol labels, fluorescent labels, and liposome or polymer sacs, are detected due to aggregation of the label. In one particular embodiment, the antibody is labeled with a colored latex particle. In an alternative embodiment, colloidal gold is used in the labeled antibody conjugate.

The label can be derivatized for linking antibodies, such as by attaching functional groups, such as carboxyl groups to the surface of a particle to permit covalent attachment of antibodies. Antibodies can be conjugated to the label using well known coupling methods. Coupling agents such as glutaraldehyde or carbodiimide can be used. The labels can be bonded or coupled to the antibodies by chemical or physical bonding. In an exemplary embodiment, a carbodiimide coupling reagent, 1-ethyl-3-(3-dimethylaminopropyl)carbodiimide (EDAC), is used to link antibodies to latex particles.

### g. Measurement of Fetal Fibronectin

Fetal fibronectin is a fetal restricted antigen found in placenta, amniotic fluid and fetal connective tissue. The presence of fetal fibronectin (fFN) in cervicovaginal secretion samples in subjects after week 12 of pregnancy is associated with a risk of impending delivery, including spontaneous abortions (12-20 weeks), preterm delivery (20-37 weeks), term (37-40 weeks) and post-date delivery (after 40 weeks), in pregnant women. In addition, the presence of fetal fibronectin in a cervicovaginal sample provides a method for determining increased risk of labor and fetal membrane rupture after week 20 of pregnancy. Detection of rupture of the amniotic membrane is important in distinguishing true and false labor, and when the rupture is small and the volume of amniotic liquid escaping is small, the rupture is often undetected. The methods and systems herein provide a means to reliably assess the risk for these conditions.

### h. Test Strip for Measuring fFN and Cellular Fibronectin

Methods for measuring fetal fibronectin and cellular fibronectin levels in cervicovaginal samples are known in the art (see, *e.g.,* U.S. Patent Nos. 5,096,830, 5,185,270, 5,223,440, 5,236,846, 5,281,522, 5,468,619 and 5,516,702), and diagnostic tests for various pregnancy-related disorders are known in the art (see, e.g., U.S. Patent Nos. 5,096,830, 5,079,171). These methods can be adapted for use with known immunoassay test strips and devices for measuring fetal fibronectin and, if desired, cellular fibronectin. Such measurements are exemplified in U.S. Patent No. 6,267,722. In particular, an immunoassay test strip for measuring fFN in cervicovaginal samples is provided therein.

### i. Antibodies for Fetal Fibronectin

An antibody that will bind the analyte of interest is conjugated to a detectable label. In a particular embodiment, where fetal fibronectin is to be detected, a mouse monoclonal anti-fFN antibody (e.g., FDC-6, as exemplified in U.S. Patent No. 5,281,522), conjugated to latex particles containing a blue dye can be used. In an alternative embodiment, a goat polyclonal antibody to human fibronectin is conjugated to a colloidal gold label.

In one embodiment, an antibody that binds the labeled antibody conjugate that is not complexed with fetal fibronectin is used as a control antibody. For example, where the labeled conjugate includes a monoclonal anti-fetal fibronectin antibody, a polyclonal goat anti-mouse IgG antibody is used.

The antibodies can be raised and purified using methods known to those of skill in the art or obtained from publicly available sources. For example, monoclonal antibody FDC-6 (deposited at the American Type Culture Collection as accession number ATCC HB 9018; see, e.g., U.S. Patent No. 4,894,326; see, also, Matsuura et al. (1985) Proc. Natl. Acad. Sci. U.S.A. 82:6517-6521; see, also, U.S. Patent Nos. 4,919,889, 5,096,830, 5,185,270, 5,223,440, 5,236,846, 5,281,522, 5,468,619 and 5,516,702), which is raised against whole molecule onco-fetal fibronectin from a tumor cell line, can be used.

### j. Fetal Fibronectin Assay Procedure

In conducting the assay, a subject sample is obtained. The sample can include fluid and particulate solids, and, thus, can be filtered prior to application to the assay test strip. The sample can be removed from the subject using a swab having a fibrous tip, an aspirator, suction or lavage device, syringe, or any other known method of removing a bodily sample, including passive methods for collecting urine or saliva. In particular, the sample can be extracted into a buffer solution, and optionally heated, for example, at 37°C and filtered. In one embodiment, where fetal fibronectin is to be detected in a sample, the sample is obtained from in the vicinity of the posterior fornix, the ectocervix or external cervical os using a swab having a dacron or other fibrous tip.

A volume of the test sample is then delivered to the test strip using any known means for transporting a biological sample, for example, a standard plastic pipet. Any analyte in the sample binds to the labeled antibody and the resulting complex migrates along the test strip. Alternatively, the sample can be pre-mixed with the labeled conjugate prior to applying the mixture to the test strip. When the labeled antibody-analyte complex encounters a detection zone of the test strip, the immobilized antibody therein binds the complex to form a sandwich complex, thereby forming a colored stripe.

Any unbound latex-conjugated antibody continues to migrate into a control zone where it is captured by a second immobilized antibody or other agent capable of binding the conjugate, and thereby forms a second colored stripe due to the aggregation of the dye-containing latex beads. This indicates that the assay run has completed.

The results of the assay are assessed using a reader and associated software. The use of point of care devices for immunoassays is known in the art, as exemplified in U.S. Patent Nos. 6,267,722 and 6,394,952. Point of care devices can provides the same or superior clinically relevant information compared to a fFN ELISA (an enzyme linked immunosorbent sandwich assay (ELISA), which is known in the art; see, e.g., U.S. Patent No. 5,281,522) test heretofore available, but in significantly less time and at the point of care. This rapid fFN immunoassay allows the user to test a cervicovaginal swab sample in about 20 minutes. When comparing the 20 minute rapid fFN test to the data from the fFN ELISA, a Kappa coefficient of 0.68 was found with a 95% confidence interval [0.62, 0.76] and an overall concordance of at least about 91.6%. These data were obtained using a system including an immunoassay test strip in combination with a reflectance reader and data processing software employing data reduction and curve fitting algorithms or neural networks, as described herein.

### k. Determining the Selection Criterion

It will be appreciated that the predetermined selection criterion will vary depending upon the particular fetal restricted antigen being determined. For example, the selection criterion can be a minimum threshold (i.e., the assay returns a positive result when the level of antigen is equal to or above the minimum threshold and a negative result when the level is below the threshold), a maximum threshold (i.e., the assay returns a positive result when the level of antigen is equal to or below the maximum threshold and a negative result when the level is above the threshold), or a range (i.e., the assay returns a positive result when the level of antigen is within the threshold range). According to one embodiment, the selection criterion tests for the presence of the fetal restricted antigen (i.e., a level of fetal restricted antigen above background). In particular, when the fetal restricted antigen being determined is fetal fibronectin, for example, a minimum threshold of about 50 ng/mL can be used.

In one embodiment, if the fetal restricted antigen assay is positive, the subject is administered a therapeutically effective amount of a progestational agent, as described herein. If, however, the fetal restricted antigen assay is negative, the subject should still be carefully monitored and repeated evaluations of the subject's fetal restricted antigen level should be performed on subsequent visits.

### 2. Determining the Level of Estriol

Estriol serves as a marker of risk of preterm delivery. The concentration of estriol in a body fluid is correlated with a standard value to determine when labor is imminent, and elevated estriol concentrations relative to the standard can indicate a risk of preterm or imminent delivery. The standard can be a predetermined range of estriol concentrations from a particular body fluid in normal pregnant subjects or a previously measured estriol concentration of the same body fluid of the same pregnant subject. In addition, increased estriol/progesterone ratios can indicate a risk of preterm or imminent delivery. The methods herein can include, but do not require, the measurement of any other substance, such as progesterone concentration in a body fluid. The methods also do not require the measurement of total estriol production over a time interval. Measurements of total estriol over a given time period, such as 24 hours, can be used with urine, if desired. Methods for measuring estriol and using measured estriol levels as an indicator of preterm or imminent delivery are known in the art, as exemplified in U.S. Pat. Nos. 5,480,776 and 5,370,135. Devices for measuring estriol concentrations are also known in the art, as exemplified in U.S. Pat. Nos. 5,786,228, 5,786,227, 5,480,776 and 5,370,135. Methods for measurement of estriol and progesterone, and using the measured ratio as an indicator of preterm or imminent delivery are known in the art, as exemplified in Darne et al., Br. Med. J. 294:270-272 (1987). An exemplary commercial product for detecting estriol is the SaIEstTM test (Biex, Dublin, CA), approved by the FDA on April 29, 1998, (FDA Application No. P970032).

The assay can be carried out on any sample of body fluid, such as blood (or a blood fraction, especially serum or plasma), urine, cervical or vaginal secretions, sweat, saliva or other fluid. Estriol is sufficiently soluble in water so that it is distributed in fluids throughout the body. Saliva can be used for simplicity of sampling and because, unlike in urine, detection is not complicated by the presence of estrogen conjugates.

Assays are generally directed to detection of unconjugated or free estriol, since conjugated estriol has reduced biological activity. In saliva about 92% of estriol is in the free form, while most estriol in urine is present as a conjugate. As will be clear to those familiar with steroid metabolism, an estriol conjugate is a compound formed by formation of a covalent linkage of a non-steroidal compound to estriol. Linkage is typically through a hydroxyl group of the steroidal ring system. The non-steroidal component can be inorganic (e.g., a sulfate group) or organic (e.g., a glucuronide group). Methods that include determining the ratio of estriol/progesterone can also be determined on the basis of unconjugated estriol/progesterone ratios, and therefore can include unconjugated estriol measurements using saliva samples.

There are no limitations on the collection and handling of samples as long as consistency is maintained. With some body fluids, such as saliva and plasma, there is little diurnal variation in estriol levels. For other fluids, notably urine, variations occur. It can be desirable to eliminate variations to the extent possible, for example by taking samples at the same time of day. Other techniques can be used to ensure consistency of measurement of analytes in clinical fluids. For example, creatinine can be measured concurrently with estriol in urine. Creatinine is produced at a constant rate in the kidneys, and measurement of creatinine concentration allows correction of volume errors in urine samples, as is well known in the art.

If desired (but not required), and depending on the source of the fluid being tested, free estriol can be separated from estriol conjugates. Techniques for such separations are known in the art. See, for example, Evan, N.Z. Med. Lab. Tech. 33:86 (1979), which describes such separations as well as two radioimmunoassays useful for measuring plasma estriol. These separations are generally difficult, and assays that do not require separation, either because of the use of specific antibodies or other binding compounds that differentiate between free and conjugated estriol, or because the sample is obtained from a source containing mostly free estriol, such as saliva can be used.

The concentration of estriol in the fluid assayed is correlated with a standard value to determine when labor is imminent. The standard is usually (1) a predetermined range of estriol concentrations for the same body fluid in normal pregnant humans in the general population, either at the corresponding time in the pregnancy or a specific time relative to normal termination of pregnancy, or (2) a previously measured estriol concentration of the same body fluid of the same pregnant human. A measured higher concentration of estriol relative to the standard value is an indication of potential onset of pre-term labor. The methods herein do not require the measurement of any other substance, such as the progesterone concentration in the body fluid, or require the measurement of total estriol production over a time interval. Measurements of total estriol over a given time period, such as 24 hours, can be used with urine, if desired.

The first general standard set out above, namely a predetermined range of estriol concentrations for the same body fluid in normal pregnant humans in general, is typically obtained by using the same assay technique that will be used in the application of the method to an individual being tested, in order to ensure the highest correlation. Sufficient measurements are made in a normal population of pregnant women to produce a statistically significant range of normal values for the value to which a comparison will be made, which typically is at preselected time intervals during normal pregnancy. While comparison to a time immediately prior to normal delivery (38 to 40 weeks) is often used, other time periods can be used. For example, estriol levels during a given week of an individual pregnancy (i.e., that of the subject) can be compared to the normal range of concentrations for the same time period (e.g., the 20th week). Generally, the minimum concentration indicative of possible onset of labor is considered to be at least 1, generally at least 2, typically at least 3 or at least 4, standard deviations above the mean estriol concentration determined just prior to the onset of labor for normal pregnant humans for any given body fluid.

It will be recognized by those familiar with statistics that the number of standard deviations used as an indication of pregnancy complications will be selected with an appropriate diagnosis goal in mind. For example, one standard deviation would encompass about 68% of normal samples; that is, 32% of normal samples would be expected to fall outside the lower and upper limits set by one standard deviation from the mean (16% would thus be expected to be above the selection limit). Thus, one standard deviation above the normal mean is not used for routine analysis, as it would include too many false positives. One standard deviation is appropriate for an assay that is desired to sweep in for further evaluation all possible candidates who might be predisposed toward pre-term labor, or this limit can be selected for subjects known to have normal or low estriol values and relatively little variation between samples. One standard deviation also can be selected for a subject known to have problems with pre-term labor in order to determine when to more closely monitor the subject under controlled conditions (such as by having a subject admitted to a hospital for constant monitoring). Two standard deviations from the mean would encompass about 95% of normal samples; three standard deviations, about 99%; four standard deviations, more than 99%. These levels are more appropriate generally, especially for subjects whose levels of estriol are known to be normal or slightly above normal or to vary from sample to sample as well as for assays with a high coefficient of variance.

It is not necessary to express the lower limit of the indication of labor (upper limit of the normal range) in standard deviations. Any other system that can be used to provide a statistically significant indication of probable onset of labor can be used. For example, the limit can be set to be a concentration that is at least as high as the 95th percentile concentration for normal subjects for the same body fluid for a normal pregnancy. In any case, a normal from the 38-42 week period, typically about 40 weeks, can selected for normal pregnancies; the concentration is monitored at 30 weeks or earlier.

Because of the many different possible clinical goals, the actual estriol level indicative of probable onset of pre-term labor is best selected by the attending physician after collecting data from several samples during the initial portion of the pregnancy and taking into consideration the time at which the measurement is being made. For example, in a normal pregnancy at week 30, the change expected in the estriol concentration prior to the onset of labor is smaller than 2 standard deviations from the mean concentration of estriol at 30 weeks. Thus, while assays in the first portion of a pregnancy (prior to 30 weeks) might use 3 or 4 standard deviations as an indication of onset of labor, two, one and a half, or even one standard deviation would be more appropriate in the later portion of a pregnancy (e.g., after 30 weeks) depending on the condition of the subject, other clinical indications in the mother known to the attending physician, and the health of the fetus. Of course, it is the earlier stages of a pregnancy that require greater attention to avoiding pre-term labor, because of the lack of fetal development at these stages and the high risk of infant death post partum. Pre-term labor is generally considered to be any labor prior to the end of a normal 40-week term of pregnancy.

The methods herein can be used for pregnancies during weeks 20 to 36, when prolonging pregnancy for even a short time is most efficacious in reducing the effects of premature birth. The assay, particularly when used to detect rate of increase, is still applicable for pregnancies terminated by labor and delivery after the end of 40 weeks, and measurements made during this time period also are contemplated herein. When applied to weeks 38 and higher, the invention is normally practiced using the "self-comparison" method discussed in more detail below; i.e., by comparing the measurement at a given time with a measurement made earlier with the same subject.

In a similar manner, subject to the same constraints discussed above, an assay concentration of at least 1, generally at least 2, typically at least 3 or at least 4, standard deviations above the mean normal concentration for the same stage of pregnancy also can be used as an indication of an abnormal pregnancy and thus as an indication of possible onset of labor, although the probability is lower if the measured level does not reach the levels considered normal for weeks 38-42.

Standard values will vary with the specific body fluid whose concentration is being measured and with the specific assay being used (although to a lesser extent). Typical minimum indicative levels of labor onset in an assay that measures unconjugated estriol are as follows for the indicated body fluids (all concentrations are in nM): saliva, at least 3, typically at least 5 or at least 6 or at least 7; serum, 30, at least 35 or at least 45.

As an alternative to comparing estriol concentrations to those present in a normal population, a previously measured estriol concentration of the same body fluid of the same pregnant human can be used as a standard for comparison. In this case, what is being determined is usually the rate of increase in estriol concentration in the fluid being tested. A positive assay (i.e., indication of imminent onset of labor) is considered to be present when the measured concentration exceeds a previously measured estriol concentration made in the same body fluid in the same pregnant human female by 50%, generally 75%, typically 100%, within one week. Again the selection of a particular rate of increase to label as the lower limit of labor onset is best selected by the attending physician for the particular reason desired. For example a screening test that is intended to collect potential problem subjects into the hospital for further observation and study could select the 50% increase as its limit in order to avoid false negative results, while accepting the problems caused by including a relatively large number of false positives. Higher percentage increases as the minimum positive indication are more acceptable for home assays and the like, in the same manner as described above for standard deviations from the normal population mean. Increases in estriol concentration that meet the standards of this paragraph and additionally reach levels previously indicated to be indicative of the onset of labor in normal populations of subjects are particularly likely to indicate imminent onset of labor.

Many assays known in the art can be used. An exemplary method is provided in U.S. Patent No. 5,480,776. In this method, an enzyme-labeled component (here a labeled estriol molecule or derivative thereof) is used in a competitive binding assay for estriol. The assay is a non-instrumented enzyme immunoassay that provides present/not-present or "threshold" (+/-) analysis results at a preselected cut-off value and thus is well adapted for use herein.

In a typical assay using this technique, the enzyme-labeled, competitive binding component comprises estriol (or the portion thereof used to generate the antibody used in the assay) bound to the immunogen that is used to produce the antibody of the assay. An enzyme label is bound to this moiety, such as through a bulky linker such as an avidin-biotin complex. The use of such a competitive binding compound allows antibodies to be used without attempting to manipulate affinity of binding of antibody to competitor while still providing the steep competitive binding curve required for a +/- analysis.

In a typical such assay, antibody is attached to a solid surface, such as a microtiter plate well, a test tube, or a porous reagent strip (such as cellulose or glass fibers). The antibody-coated solid surface is then contacted simultaneously with a sample and with a competitive binding compound. By providing fewer antibody binding sites than are present in the combined total of analyte and competitive binding compound, only a fraction of the molecules in solution will bind to the solid surface. If there are no analyte molecules present, all of the binding sites will be taken up by the competitive binding compounds so that a maximum amount of enzyme is attached to the solid surface. When a substrate for the enzyme is contacted with the solid surface after the sample is washed away, reaction of the enzyme with the substrate provides a detectable signal (usually formation of a color) that indicates to the user the absence of analyte in the sample (a negative result). If analyte is present in the sample, analyte competes for binding sites so that less of the enzyme-labelled competitor can bind. By using a bulky binding composition, which binds less rapidly to the antibody than does the analyte, and by properly selecting the number of binding sites relative to the amount of sample added (which is a standard technique to one of skill in the art), analyte present at a concentration above a preselected minimum level will exclude binding of the competitive binding composition and thus binding of the enzyme to the solid substrate. An example of such a selection process to provide different threshold levels is set out in the cited patent application for estradiol. The same selection process can be used with estriol to carry out an assay as described herein. Thus, if sufficient analyte is present in the sample, after reaction no enzyme is present to produce a color change and the reaction mixture stays the same (thus a positive reaction using this reaction scheme).

Other reaction schemes can be used in which the formation of color is indicative of the presence of the analyte. The previous example is merely one of many types of competitive binding assays in which estriol can be measured.

Antibody production for use in an assay for estradiol is conventional and is not described here in detail. A brief discussion of general techniques for the production of antibodies specific for steroids follows.

An animal is injected with a composition containing estriol covalently attached to an immunogen, usually a protein, prepared as described above. Multiple injections or the use of an adjuvant will ensure maximum stimulation of the immune system and production of antibodies. If polyclonal antibodies are desired, they can be prepared by simply collecting blood from the immunized animal and separating the antibodies from other blood components by standard techniques. To obtain monoclonal antibodies, the spleen or lymphocytes from the immunized animal are removed and immortalized or used to prepare hybridomas by cell-fusion methods known to those skilled in the art. Antibodies secreted by the immortalized cells are screened to determine the clones that secrete antibodies of the desired specificity. For monoclonal anti-estriol antibodies, the antibodies must bind to estriol. Cells producing antibodies of the desired specificity are selected, cloned, and grown to produce the desired monoclonal antibodies.

Antibody can be attached to a solid surface for use in an assay using known techniques for attaching protein material to solid support materials. The solid support can include plastic surfaces of test tubes or microtiter plates, polymeric beads, dip sticks, or filter materials. The attachment methods include non-specific adsorption of the protein to the support and covalent attachment of the protein, typically through a free amino group, to a chemically reactive group on the solid support, such as an activated carboxyl, hydroxyl, or aldehyde group.

### 3. Determining the Level of Insulin-like Growth Factor Binding Protein One

In one embodiment, when fetal fibronectin is determined to be at level or in amount indicative of a risk of preterm or imminent delivery, an assay of insulin-like growth factor binding protein one (IGFBP-1) can be performed on a sample from the subject to determine whether the membranes are intact. The cervicovaginal sample can be the same or different from the sample used to assay for the fetal restricted antigen. Methods for performing the IGFBP-1 assay are known in the art, as exemplified in international publication No. WO 94/17405, and U.S. Pat. Nos. 5,597,700 and 5,968,758. If the IGFBP-1 assay is negative for the presence of IGFBP-1, the membranes have not ruptured and the subject can be administered a progestational agent, as described herein. If, however, the IGFBP-1 assay is positive indicating that the membranes have ruptured, the test indicates that delivery cannot be delayed and the progestational agent should not be administered.

IGFBF-1 is assayed by any quantitative or semi-quantitative procedure that can either determine the amount of IGFBP-1 in the sample or that the amount of IGFBP-1 is above a threshold amount that indicates rupture of membranes.

Anti-IGFBP-1 antibodies can be produced by a number of methods. Polyclonal antibodies can be induced by administering an immunogenic composition comprising human IGFBP-1 to a host animal. Alternatively, amniotic fluid or another source of high levels of IGFBP-1 can be used as the immunogen and antibodies of the desired specificity can be identified.

Preparation of immunogenic compositions of IGFBP-1 can vary depending on the host animal and is well known. For example, IGFBP-1 or an antigenic portion thereof can be conjugated to an immunogenic substance such as KLH or BSA, or provided in an adjuvant or the like. The induced antibodies can be tested to determine whether the composition is IGFBP-1-specific. If a polyclonal antibody composition does not provide the desired specificity, the antibodies can be purified to enhance specificity by a variety of conventional methods. For example, the composition can be purified to reduce binding to other substances by contacting the composition with IGFBP-1 affixed to a solid substrate. Those antibodies which bind to the substrate are retained. Purification techniques using antigens affixed to a variety of solid substrates such as affinity chromatography materials including Sephadex, Sepharose and the like are well known.

Monoclonal IGFBP-1-specific antibodies also can be prepared by conventional methods. A mouse can be injected with an immunogenic composition comprising IGFBP-1, and spleen cells obtained. Those spleen cells can be fused with a fusion partner to prepare hybridomas. Antibodies secreted by the hybridomas can be screened to select a hybridoma wherein the antibodies react with IGFBP-1 and exhibit substantially no reaction with the other proteins which can be present in a sample. Hybridomas that produce antibodies of the desired specificity are cultured by standard techniques. Hybridoma preparation techniques and culture methods are well known.

The assay conditions and reagents can be any of a variety found in the prior art. The assay can be heterogeneous or homogeneous, conveniently a sandwich assay. The assay usually employs solid phase-affixed anti-IGFBP-1 antibodies. The antibodies can be polyclonal or monoclonal or suitable antibody fragments or other binding moieties. The solid phase-affixed antibodies are combined with the sample. Binding between the antibodies and sample can be determined in a number of ways. Complex formation can be determined by use of soluble antibodies specific for IGFBP-1. The antibodies can be labeled directly or can be detected using labeled second antibodies specific for the species of the soluble antibodies. Various labels include radionuclides, enzymes, fluorescers, colloidal metals or the like. Conveniently, the assay will be a quantitative enzyme-linked immunosorbent assay (ELISA) in which antibodies specific for IGFBP-1 are used as the solid phase-affixed andenzyme-labeled, soluble antibodies. Alternatively, the assay can be based on competitive inhibition, where IGFBP-1 in the sample competes with a known amount of IGFBP-1 for a predetermined amount of anti-IGFBP-1 antibody. For example, any IGFBP-1 present in the sample can compete with a known amount of the labeled IGFBP-1 or IGFBP-1 analogue for antibody binding sites. The amount of labeled IGFBP-1 affixed to the solid phase or remaining in solution can be determined.

Appropriate dilution of the conjugate can be performed to detect the selected threshold level of IGFBP-1 which is above background values for the assay as a positive sample.

The assay results can be interpreted as follows. IGFBP-1 levels below 20-50 ng/mL are considered background and are negative. The cut-off of choice for the background level depends upon whether a high sensitivity or high specificity test is desired. For example, as described in the examples, when 42 cervicovaginal secretion specimens which exhibited a positive fetal fibronectin test (> 50 ng/mL) for impending delivery and were ferning, pooling, and nitrazine negative for rupture of membranes were tested for IGFBF-1, one of these specimens demonstrated 42 ng/mL IGFBP-1. If a cut-off of 20 ng/mL were to be used, the demonstrated specificity of the test to rule out rupture would be 97%. On the other hand, if 50 ng/mL were to be used, the rule out specificity of the test would be 100%. In most cases, high rule-out specificity can be used, since subjects with rupture of membranes are in greater danger of infection than those who do not have rupture, so a cutoff is 20-50 ng/mL.

The cutoff of 20-50 ng/mL was determined for the assay described in the examples. As is well known, other assays using different reagents can have different cutoff values. For example, IGFBP-1 antibodies which differ in their antigen binding characteristics can produce assay results with different optimal cut off values. One of ordinary skill will recognize that background values can vary when different reagents are used and will understand how to determine the proper background level for the desired specificity and sensitivity for a selected assay.

The presence of IGFBP-1 in a cervicovaginal secretion sample from a subject who is positive for a marker that indicates increased risk of delivery indicates that the membranes have ruptured. If IGFBP-1 is less than 20-50 ng/mL or undetectable (background for the assay), the membranes remain intact. When IGFBP-1 is positive (> 20-50 ng/mL) and the delivery marker (e.g., fetal fibronectin) is negative, then amniotic membranes can have ruptured, although most subjects who have ruptured membranes will exhibit positive IGFBP-I and the delivery marker simultaneously. When IGFBP-1 is negative, the IGFBP-1 test can be repeated, typically daily, until the sample is positive for IGFBP-1 .

In addition to, or as an alternative to, the IGFBP-1 assay, the concentration of estriol can be determined in a sample obtained from the subject. Methods for determining the level of estriol in a sample that can be adapted for use in the present methods are known in the art, as demonstrated, for example, in U.S. Patent No. 5,480,776, issued January 2, 1996. There are no limitations on the type of assay used to measure estriol. Any of the current assays for estriol can be used, as well as assays that can be developed in the future. Examples of estriol assays are described in detail below.

### D. Administering the Progestational Agent

The progestational agent can be administered in any of a variety of conventional forms. For example, the progestational agent can be administered orally, parenterally by injection (e.g., by bolus injection or continuous infusion), transdermally, intranasally, or by inhalation.

It will be appreciated that the therapeutically effective amount of progestational agent will vary according to, for example, the particular agent and/or pharmaceutical composition being used, the mode of administration, and the course of treatment. Optimal dosages for a given set of conditions can be ascertained using conventional dosage-determination tests. Further, administration of the progestational agent can be repeated at appropriate intervals (e.g., daily, weekly, etc.). In one embodiment, the dose is determined by measuring the concentration of progestational agent in the circulating blood and adjusting the mode of administration and/or course of treatment accordingly.

When the progestational agent is 17-α-hydroxyprogesterone caproate administered intramuscularly, for example, the agent can be administered weekly at a dose of at least about 100 mg/week, at least about 250 mg/week, at least about 500 mg/week, or at least about 1000 mg/week. Alternatively, when the progestational agent is 17-α-hydroxyprogesterone caproate administered intramuscularly, the agent can be administered daily at a dose of at least about 10 mg/day, at least about 25 mg/day, at least about 80 mg/day, at least about 100 mg/day, or at least about 200 mg/day.

The progestational agent is administered for a period of time sufficient to obtain the desired benefits of the progestational agent. The progestational agent can be administered after 20 weeks, gestation, after about 28 weeks gestation, or after about 35 weeks gestation. Administration of the progestational agent is optionally stopped at about 36 weeks gestation or at the onset of spontaneous labor.

### E. Combinations and Kits

Combinations and kits containing the combinations also are provided. Combinations include an anti-(preterm delivery marker) antibody and a progestational agent. Anti-(preterm delivery marker) antibodies of such combinations include an anti-(fetal restricted antigen) antibody or an anti-(fetal restricted antigen class) antibody or any combination thereof. The combinations can include one or more anti-(preterm delivery marker) antibodies immobilized to a solid support. The combinations can include two or more of the same anti-(preterm delivery marker) antibodies. For example, a combination can include FDC-6 conjugated to colloidal gold and can also include FDC-6 immobilized to a solid support. Combinations can include one or more reagents for detecting an anti-(preterm delivery marker) antibody. The combination also optionally contains a device for administering the progestational agent, such as, for example, a syringe. Combinations can also include one or more anti-(membrane rupture marker) antibodies such as an anti-IGFBP-1 antibody.

Kits are packaged combinations that optionally include instructions and/or other reagents or devices. A kit can include a device for obtaining a sample from the subject. For example, the kit can contain a vaginal sample collection device such as a dacron swab, a vaginal sample filtration device, and/or a vessel containing a vaginal sample diluent. In another example, the kit can contain a saliva sample collection device. Kits can also include one or more reagents such as buffers for stabilizing the sample and/or reagents for detecting the presence of an anti-(preterm delivery marker) antibody. Kits can include filters or compositions that remove background material and/or enhance detection of a preterm delivery marker. The combinations and kits optionally including instructions for collecting the sample and/or performing the assay. A variety of combinations and kits are known in the art which can be adapted for use in the methods provided herein. Such known kits are exemplified in U.S. Patent Nos. 5,281,522, 6,394,952, and 6,267,722.

### F. Reference examples

The following examples are included for illustrative purposes only and are not intended to limit the scope of the invention.

### EXAMPLE 1: Polyclonal Anti-(fetal fibronectin) Antibody

Fetal fibronectin is purified from amniotic fluid as described by Engall and Ruoslahti, Int. J. Cancer 20:1-5 (1977).

The anti-(fetal fibronectin) antibodies are elicited in rabbits using the immunization techniques and schedules described in the literature, e.g., Stollar, Meth. Enzym. 70:70 (1980), immunizing the rabbits with the fetal fibronectin antigen. The antiserum is screened in a solid phase assay similar to that used for monoclonal antibodies, e.g., as described by Lange et al, Clin. Exp. Immunol. 25:191 (1976) and Pisetsky et al, J. Immun. Meth. 41:187 (1981).

The IgG fraction of the antisera is purified further by affinity chromatography using CNBr-Sepharose 4B (Pharmacia Fine Chemicals) to which has been coupled fetal fibronectin. The method used for coupling is that recommended by the gel manufacturer, AFFINITY CHROMATOGRAPHY. Pharmacia Fine Chemicals, pp 15-18.

The column is equilibrated with from 2 to 3 volumes of buffer (0.01 M PBS, pH 7.2), and the anti-(fetal fibronectin) antibody containing solution is then applied to the column. The absorbency of the eluate is monitored at 280 nm until protein no longer passes from the column. The column is then washed with 0.1 M glycine buffer, pH 2.5, to desorb the immunoaffinity bound anti-(fetal fibronectin) antibody. Peak protein fractions are collected, pooled and dialyzed against 0.01 M PBS, pH 7.2, for 24-36 hr at 4° C. with multiple buffer changes.

If a higher purity is desired, the affinity purified IgG can be passed through an adult plasma fibronectin bound affinity column by the procedure described above to remove any antibodies which would cross-react with adult plasma fibronectins.

### EXAMPLE 2: Monoclonal Anti-(fetal fibronectin) Antibody

Using the purified fetal fibronectin obtained by the procedure of Example 1, mouse monoclonal antibodies to the fetal fibronectin are obtained using standard procedures of Galfre and Milstein, Meth. Enzym. 73:1 (1981) and Matsuura, H. and Hakomori, S. et al, Proc. Natl. Acad. Sci. USA 82:6517-6521 (1985), using fetal fibronectin as the antigen for immunizing the mice. The monoclonal antibodies are screened using a modification of the techniques described in the literature, e.g., Lange et al, Clin.Exp.Immunol. 25:191 (1976) and Pisetsky et al, J. Immun. Meth. 41:187 (1981).

Mouse monoclonal antibody is purified from ascites fluid or from hybridoma culture supernatants using Protein-A coupled Sepharose-4B (Pharmacia Fine Chemicals) according to the procedure of Tijsson, PRACTICE AND THEORY OF ENZYME IMMUNOASSAYS. Elsevier Science Publishers, pp 105-107 (1985).

### EXAMPLE 3: Polyclonal Anti-(fetal fibronectin) Antibody-Coated Microtiter Plate

Rabbit anti-(fetal fibronectin) prepared and further purified to remove adult fibronectin cross-reactivity as described in Example 1 is diluted to 10 µg/mL in 0.05 M carbonate buffer, pH 9.6. 100 µL is dispersed into each well of an IMMULON II microtiter plate (Dynatech). The plate is covered and incubated 4 hr at room temperature or 4° C. overnight. The plate is washed 4 times with Wash Buffer (0.02 M Tris HCl, 0.015 M NaCl, 0.05% TWEEN-20), filling and emptying the wells completely with each use. The plate is then blocked by dispersing into each well 200µL of a blocking solution (0.01 M PBS, 1% BSA, 0.02% NaN₃, pH 7.4) and incubating for 1 hr at room temperature. The wells are then washed 4 times with Wash Buffer, as described above. The plate is now ready for immunoassay of samples.

### EXAMPLE 4: Polyclonal Anti-Human Fibronectin Antibody

Human plasma fibronectin was purified from human plasma as described by Engvall and Ruoslahti, Int. J. Cancer 20:1-5 (1977).

The anti-human plasma fibronectin antibodies were elicited in goats using the immunization techniques and schedules described in the literature, e.g., Stollar, Meth. Enzym. 70:70 (1980), immunizing the goats with the human plasma fibronectin antigen. The antiserum was screened in a solid phase assay similar to that used for monoclonal antibodies, e.g., as described by Lange et al, Clin. Exp. Immunol. 25:191 (1976) and Pisetsky et al, J. Immun. Meth. 41:187 (1981).

The IgG fraction of the antiserum was purified further by affinity chromatography using CNBr-Sepharose 4B (Pharmacia Fine Chemicals) to which has been coupled human plasma fibronectin according to the method recommended by the manufacturer (AFFINITY CHROMATOGRAPHY, Pharmacia Fine Chemicals Catalogue 1990), pp 15-18.

Briefly, the column was equilibrated with from 2 to 3 volumes of buffer (0.01 M PBS, pH 7.2), and the anti-human fibronectin antibody-containing solution was then applied to the column. The absorbency of the effluent was monitored at 280 nm until protein no longer passed from the column. The column was then washed with equilibration buffer until a baseline absorbance at 280 nm was obtained.

The immunoaffinity bound anti-human plasma fibronectin antibody was eluted with 0.1 M glycine buffer, pH 2.5. Peak protein fractions were collected, pooled and dialyzed against 0.01 M PBS, pH 7.2, for 24-36 hr at 4°C. with multiple buffer changes. The above procedure was repeated to immunize rabbits with human plasma fibronectin and to purify the resultant polyclonal anti-human fibronectin antibodies.

### EXAMPLE 5: Polyclonal Anti-Fibronectin Antibody-Coated Microtiter Plate

Goat anti-human plasma fibronectin prepared as described in Example 4 is diluted to 10 µg/mL in 0.05 M carbonate buffer, pH 9.6. 100 µL is dispersed into each well of a polystyrene microtiter plate such as supplied by Costar, Nunc, or Dynatech. The plate is covered and incubated 2 to 4 hr at room temperature or 4° C. overnight. The plate is washed 3 to 4 times with Wash Buffer (0.02 M Tris HCl, 0.015 M NaCl, 0.05% TWEEN-20), filling and emptying the wells completely with each use. The plate is then blocked by dispersing into each well 200 µL of a blocking/stabilizing solution (4% sucrose, 1 % mannitol, 0.01 M PBS, 1 % BSA, 0.02% NaN₃, pH 7.4) and incubated for 30 minutes to 2 hrs at room temperature. The wells are then aspirated to dryness, the plate is packaged in an air-tight container with a desiccant pouch, and stored at 4° C. until needed.

### EXAMPLE 6: Monoclonal Antibodies from Hybridoma HB 9018

Known methods for preparation of the Hybridoma deposited at the American Type Culture Collection and given the accession number ATCC HB 9018 were carried out, as exemplified in U.S. Patent No. 4,894,326 issued Jan. 16, 1990 to Matsuura et al.

The hybridoma was cultured by growth in RPMI 1640 tissue culture medium supplemented with 10% fetal bovine serum. Additionally, the hybridoma was cultured in mice by the injection of the hybrid cells according to the procedure of Mishell and Shiigi (Selected Methods in Cellular Immunology, W.H. Freeman & Co, San Francisco p 368, 1980).

The monoclonal antibody designated FDC-6 and produced by the hybridoma was prepared for use in an immunoassay by the following procedure. The IgG fraction of the culture supernatant or the ascites was precipitated by ammonium sulfate fractionation. The antibody was redissolved and dialyzed into the appropriate buffer for purification by affinity chromatography on Protein-G Fast Flow (Pharmacia Fine Chemicals) according to the manufacturer's directions.

### EXAMPLE 7: Monoclonal Antibody-Coated Microtiter Plate

Microtiter plates were coated with FDC-6 monoclonal antibody by following the procedure described below.

Monoclonal antibody FDC-6 prepared as described in Example 6 was diluted to 10 µg/ml in phosphate buffer, pH 7.2 and 100 µl/well was dispersed into a polystyrene microtiter plate (Costar). The plates were incubated for 2 hours at room temperature or overnight at 4° C. The contents of the wells were aspirated and the wells washed 3 to 4 times with wash buffer (0.02 M Tris HCl, 0.015 M NaCl, 0.05% TWEEN-20) as described in Example 5. 200 µl/well of blocking/stabilizing solution (4% sucrose, 1% mannitol, 0.5% casein, 0.01 M PBS) was then added to the wells and incubated for 30 minutes to 4 hours at room temperature. The wells were then aspirated to dryness, and the plate was packaged in an air-tight container with a desiccant pouch, and stored at 4° C. until needed.

The above procedure was repeated using microtiter plates from Nunc and Dynatech and gave equivalent results.

### EXAMPLE 8: Enzyme Labeled Anti-(fibronectin) Antibody

Anti-human plasma fibronectin antibody prepared according to Example 4 was conjugated with alkaline phosphatase following the one-step glutaraldehyde procedure of Avrameas, Immunochem. 6:43 (1969).

### EXAMPLE 9: Fetal Fibronectin Assay Kit and Method

An assay kit for the fetal restricted antigen, fetal fibronectin included the following reagents:
1. a microtiter plate coated with murine monoclonal anti-fetal fibronectin antibody.
2. alkaline phosphatase-conjugated, affinity purified, polyclonal, goat anti-fibronectin antibodies,
3. enzyme substrate
4. a negative control
5. a positive control
6. rinse buffer concentrate (50X)

The microtiter plate coated with murine monoclonal anti-fetal fibronectin antibody and the alkaline phosphatase-conjugated, affinity purified, polyclonal, goat anti-fibronectin antibodies were prepared as described in Examples 7 and 8, respectively. The microtiter plate was packaged as 12 strips of eight wells each in sealed plastic bags containing desiccant.

The stock antibody conjugate was appropriately diluted in conjugate diluent (0.05 M Tris Buffer pH 7.2, 2% D-Sorbitol, 2% BSA, 0.1 % Sodium Azide, 0.01 % Tween-20, 1 mM Magnesium Chloride, and 0.1 % Zinc Chloride) and 10 ml placed in a polyethylene dropper bottle container.

The enzyme substrate (10 mL in a polyethylene dropper bottle container) was phenolphthalein monophosphate (1mg/ml) dissolved in 0.4 M aminomethylpropanediol buffer, pH 10 with 0.1 mM magnesium chloride and 0.2% sodium azide.

The positive control (2.5 mL in a polyethylene dropper bottle container) was amniotic fluid containing fetal fibronectin diluted to a concentration of fetal fibronectin of 50 ng/mL in sample diluent solution (0.05 M Tris buffer pH 7.4, 1 % bovine serum albumin (BSA), 0.15 M sodium chloride, 0.02% Sodium Azide, 5 mM ethylenediamine tetraacetic acid (EDTA), 1 mM phenylmethylsulfonyl fluoride (FMSF), and 500 Kallikrein Units/ml of Aprotinin). This sample diluent solution is described in U.S. Patent No. 4,919,889 to Jones et al, issued Apr. 24, 1990.

The negative control (2.5 mL in a polyethylene dropper bottle container) was the sample diluent solution used for the positive control without fetal fibronectin.

The rinse buffer (10 mL in a polyethylene dropper bottle container) was a 50X concentrate containing 1.0 M Tris buffer pH 7.4, 4.0 M sodium chloride, 2.5% Tween-20, and 1% sodium azide. The rinse buffer was diluted with water to a final concentration of 0.02 M Tris, 0.08 M sodium chloride, 0.05% Tween-20, and 0.02% sodium azide for use in the assay.

In addition, 5µm pore size polyethylene sample filters (Porex Technologies, Fairburn, Ga.), a microtiter strip holder, a microtiter plate cover and an instruction sheet. All of the dropper bottles in the kit were polyethylene bottles designed to dispense approximately 50 µL drops of the reagent. All of the assay steps performed following sample collection utilized the reagents and materials in the kit.

The assay was performed as follows. All samples were collected in the vicinity of the posterior fornix or cervical os using dacron swabs. Swab samples were immersed in 1.0 mL of sample diluent in a collection vial. The sample diluent solution is described above. The swabs were removed from the solution leaving as such liquid as possible in the collection tube. The samples were incubated at 37° C. along with the controls from the assay kit for 15 minutes prior to the assay, either before or after filtration. A sample filter was snapped in place on each sample tube. The 8-well strips were snapped into place in a strip holder. The holder had the alphanumeric indications of the 12 columns and eight rows of standard microtiter plates. Duplicate 100µL aliquots of each sample and the positive and negative controls were placed in separate wells of the microtiter strip and incubated for 1 hour at room temperature.

Following incubation, samples and controls were aspirated from the wells. Wells were washed three times with diluted wash buffer (1X). Following washing, 100µL of enzyme-antibody conjugate was added to each well and incubated for 30 minutes at room temperature. The wells were aspirated and washed as described above. Following washing, 100 µL of enzyme substrate was added to each well and incubated for 30 minutes at room temperature.

Following the incubation, the plates were gently agitated by hand or with an orbital shaker to mix the well contents. The frame of strips was placed in an ELISA plate reader. The absorbance of each well at 550 nm was determined. The average absorbance of the duplicate wells for each sample and control was calculated. If the absorbance of the subject sample was less than the absorbance of the positive control, the sample was negative, indicating an undetectable level of fetal fibronectin in the sample. If the sample absorbance is greater than or equal to the absorbance of the positive control, the sample was positive, indicating that fetal fibronectin was present in the sample. In any assay if the absorbance of the positive control was not greater than 1.5 times the absorbance of the negative control the results were discarded and the assay procedure was repeated.

### EXAMPLE 10: Preterm Labor Sandwich Immunoassay

The procedure of Example 9 was repeated with test samples obtained during weeks 20-36 of pregnancy. Studies were conducted at three perinatal referral clinics in the United States. Women were evaluated for admission to the hospital for either suspected preterm rupture of membranes or suspected preterm labor with intact membranes.

Confirmation of rupture of membranes was made by visual examination of the vagina for gross pooling of amniotic fluid, microscopic examination of dried vaginal secretions for ferning, presence of alkaline vaginal secretions using nitrazine paper and ultrasound diagnosis of oligohydramnios. Rupture of membranes was defined by the presence of any two of these four diagnostic criteria. One hundred-seventeen women with intact amniotic membranes pregnant between 23 weeks and 36 weeks, 6 days of gestation based on last known menstrual period and expected date of confinement confirmed by first trimester pelvic examination and ultrasonography <28 weeks gestation are subsequently described. Women were determined by the attending physician to be at risk for preterm labor and subsequent delivery based on medical history and clinical examination including recording of uterine contractions and examination of the cervix. Since the clinical definition of preterm labor is sometimes difficult to establish, data establishing the clinical utility of fetal fibronectin were analyzed using preterm delivery as the outcome variable.

To assess the potential for cervicovaginal contamination by maternal plasma fibronectin, maternal blood specimens were obtained from 52 women with apparently healthy pregnancies during second or third trimester. Amniotic fluid specimens were obtained from 92 subjects undergoing amniocentesis for genetic diagnosis in early second trimester and 8 subjects undergoing amniocentesis for evaluation of fetal lung maturity prior to elective repeat, cesarean section in third trimester.

The assay results indicated that the concentration of fetal fibronectin in amniotic fluid in second trimester was 87.1 ±4.8 µg/ml (n = 92) and 27.1 ± 17.3 µg/ml (n = 8) in third trimester. The concentration of fetal fibronectin in maternal plasma in the second trimester was 1.48 ± 0.11 µg/ml (n = 20) and 3.19 ± 0.30 µg/ml (n = 32) in the third trimester.

As is shown in the table above for the 117 subjects with suspected preterm labor and intact amniotic membranes, 49 of 59 (sensitivity = 83.1%) women delivering prematurely (PTD) had fetal fibronectin in their cervicovaginal secretions compared to 11 of 58 women (specificity=81.0%) delivering at term (TD) (p<0.01). Similarly, those subjects with fetal fibronectin in their cervicovaginal secretions were far more likely to deliver prematurely (positive predictive value =81.7%) than those women not expressing cervicovaginal fetal fibronectin (negative predictive value=82.5%).

The presence of cervicovaginal fetal fibronectin was a sensitive and specific predictor of the risk for preterm delivery in these women with suspected preterm labor. The presence of fetal fibronectin in these subjects was strongly associated with risk of preterm delivery with a logistic regression odds ratio of 3.79 (95% Cl:2.33, 6.15; p<0.01).

To evaluate for potential confounding by fetal fibronectin of maternal origin, the data was analyzed after exclusion of 31 samples contaminated with blood. As shown below, similar proportions of subjects had fetal fibronectin in their cervicovaginal secretions and delivered prematurely. Furthermore, inclusion of the presence or absence of vaginal bloody show into the stepwise logistic regression model gave an odds ratio of 1.70 (95%Cl: 0.91,3.18; p=0.1) demonstrating that bloody show was not an independent predictor of preterm delivery after fetal fibronectin was introduced into the model. It was clear, however, from univariate analysis that detection of fetal fibronectin in cervicovaginal secretions contaminated with blood is an indicator of imminent delivery.

The utility of fetal fibronectin for identifying women at risk for PTD was maintained even when women in preterm contractions with intact membranes with cervical dilation exceeding 2 cm were eliminated from the analysis. The logistic regression odds ratio of 3.18 (95%Cl: 1.8,5.6, p <0.01) confirmed the predictive value of fetal fibronectin in this clinically discrete population.

### EXAMPLE 11 : Fetal Fibronectin Assay Kit and Method

An assay kit for the fetal restricted antigen, fetal fibronectin included the following components. This kit was designed to be used to perform a rapid, bedside assay.
1. an assay device comprising a plastic housing and containing:
   (a) a porous nylon membrane to which is bound a monoclonal anti-fetal fibronectin antibody;
   (b) a flow control membrane system; and (c) an absorbent layer
2. a colloidal gold-labeled goat anti-fibronectin antibody conjugate in a protein matrix
3. conjugate reconstitution buffer
4. a wash solution
5. a sterile, dacron sample collection swab

The membrane device was prepared by the following procedure. Approximately 2µL of the murine monoclonal antibody FDC-6 prepared as described in Example 6 is applied to a membrane surface (1.2µm nylon, Biodyne-A, Pall) in a pH 6, 0.01 M phosphate buffered saline (PBS), 0.1 M citrate buffer containing 0.5 mg/ml BSA. A procedural control consisting of human plasma fibronectin purified as described in Example 4 in the same buffer also is applied to a discrete region of the membrane. After the membrane has air dried, a blocking reagent of PBS-buffered, 0.5% nonfat dry milk is added to the membrane. The excess blocking reagent is removed after at least about 20 minutes.

The membrane-holding device (Target Device, V-Tech, Pomona, Calif.) is assembled with a second porous layer (0.45µm low protein-binding nylon, LoProdyne, Pall) beneath the antibody-bearing membrane (in the direction of sample application) for controlling the flow of sample solution from the assay membrane to the absorbent layer. The two porous membranes are then placed over an absorbent porous polyethylene layer having a capacity of greater than 1.5 ml (Chromex, Brooklyn, N.Y.) and enclosed in the device. The device is packaged individually in a sealed plastic bag containing desiccant.

The colloidal gold is prepared by the reduction of 0.01 % tetrachioroauric acid with 0.16% sodium citrate in a manner which produces approximately 30 nm particles. Briefly, the two solutions are heated separately to 90° C. The reducing solution is added to the gold solution while vigorously stirring. The combined solution is boiled (100° C) for at least 10 minutes.

Affinity purified goat anti-fibronectin antibody (prepared as described in Example 4) was bound to the colloidal gold by adsorption. Briefly, the colloidal gold solution prepared above was combined with the antibody (5-10 µg/mL) in water. Following conjugation, the conjugate solution was stabilized by the addition of 5% BSA and 5% polyvinylpyrrolidine (final concentration).

The stock conjugate was concentrated approximately 10- to 12-fold by ultrafiltration using a hollow fiber filter. The concentrated conjugate was diluted to an appropriate level in 15 mM Tris, 2% BSA, 0.1% Tween 20, 0.2% polyethylene glycol, 8% polyvinylpyrrolidine and 0.04% thimerosal. An appropriate concentration was determined by using a range of dilutions in a sample assay procedure as described below and determining the dilution which produces the best result.

The selected conjugate dilution is placed in polyethylene sample collection tubes and lyophilized. The tubes are fitted with 2µm pore size polyethylene sample filters (Porex Technologies, Fairburn, Ga.) during the lyophilization process. The lyophilized conjugate is individually packaged in a foil pouch with desiccant.

The conjugate reconstitution buffer is 100 mM sodium acetate. This buffer is packaged as a unit dose in a 1 ml disposable tube.

The wash solution is water packaged as a unit dose in a disposable tube.

The kit additionally contains an individually packaged sterile dacron swab and a procedural summary card.

The assay was performed as follows:
1. Before collecting the sample, remove the plastic tube containing gold conjugate from the foil pouch, remove the dropper tip and add the entire contents of the tube containing the conjugate reconstitution buffer.
2. Collect the sample with the swab provided. During a sterile speculum examination, insert the swab into the posterior fornix of the vagina, twirl for approximately 10 seconds to absorb fluid. Immediately proceed to perform the test. Samples cannot be stored for later testing. Place the swab in the gold conjugate solution and mix rapidly with an up and down motion for 10 to 15 seconds.
3. Remove as much liquid as possible from the swab by rolling the tip on the inside of the tube. Dispose of the swab in a manner consistent with handling potentially infectious materials.
4. Replace the dropper tip on the plastic tube and immediately dispense the entire volume of diluted filtered sample onto the surface of the membrane device.
5. After the sample liquid has been absorbed into the membrane surface, add a few drops of wash solution and observe the results.
6. A negative result is indicated by a red color in the procedural control area of the membrane only. A positive result is indicated by a pink or red spot in the test zone of the membrane as well as in the control zone.

### EXAMPLE 12: Detection of IGFBP-1

### IGFBP-1 was detected by the procedure described below.

### Preparation of Anti-IGFBP-1 Monoclonal Antibodies

A panel of hybridomas was generated by immunization of mice with human amniotic fluid. One monoclonal antibody (designated AF127) reacted with a 31 kd protein which was found to be one of the most abundant proteins of amniotic fluid. A two dimensional gel Western blot was used to identify the polypeptide antigen. This protein was so abundant in baboon amniotic fluid, that the protein was transferred to polyvinylidene difluoride membrane, which is a suitable absorbant for determining the sequence of a protein.

The N-terminus of the protein was sequenced using a single paper disk punched out with a conventional paper hole puncher containing 100 picomoles protein amino acid by Edman degradation amino acid sequencer using an Applied Biosystems, Inc. Model 477A amino acid sequencer. The N-terminal sequence was determined to be APWQCAPCSAEKLALPPVPASCSEVTRSA, (SEQ ID NO.1) which identified the protein as IGFBP-1 using GenBank.

The monoclonal antibody designated AF127 and produced by the hybridoma was prepared for use in an immunoassay by the following procedure. The IgG fraction of the culture supernatant or the ascites was purified using Avid Al affinity gel purification for immunoglobulins, according to the manufacturer's directions (Bioprobe International, Inc. Tustin, CA).

### Preparation of Anti-IGFBP-1-Coated Microtiter Plate

Microtiter plates were coated with IGFBP-1 monoclonal antibody by the procedure described below.

Monoclonal antibody IGFBP-1 prepared as described above was diluted to 10 µg/mL in PBS (0.01 M phosphate buffer, 0.15 M NaCl, pH 7.4, 0.02% NaN₃), and 100 µL/well was dispersed into a polystyrene microtiter plate (Costar). The plates were incubated overnight at 40 °C. The contents of the wells were aspirated and the wells washed once with wash buffer (0.02 M Tris HCl, pH 7.9, 0.15 M NaCl). 250 µl/well of blocking solution (3% IGFBP-1-free BSA in PBS) was then added to the wells and incubated for 2 hours at room temperature. The wells were aspirated and then washed once as described above and stored.

### Preparation of Polyclonal Anti-IGFBP-1 Antibodies

IGFBP-1 was purified from baboon amniotic fluid using gel electrophoresis followed by electroelution/electrotransfer. The anti-baboon IGFBP-1 antibodies were elicited in goats using the standard immunization techniques and schedules, by immunizing the goats with the baboon amniotic fluid (which contained IGFBP-1).

The antiserum was screened in a solid phase assay similar to that used for monoclonal antibodies, e.g., as described by Lange et al, Clin. Exp. Immunol. 25:191 (1976) and Pisetsky et al, J. Immun. Meth. 41:187 (1981).

### Assay Reagents

### The assay was performed using the following additional reagents.

Commercially available swine anti-goat alkaline phosphatase antibody conjugate (TAGO, Burlingame, California) was appropriately diluted in conjugate diluent (0.02 M Tris buffer, pH 7.9, 1 % BSA, 0.1% sodium azide, 0.05% TWEEN-20). The enzyme substrate was phenolphthalein monophosphate (1 mg/mL) dissolved in 0.4 M aminomethylpropanediol buffer, pH 10 with 0.1 mM MgCl₂ and 0.2% sodium azide.

The positive control was human amniotic fluid diluted to a concentration of IGFBP-1 of 50 ng/mL in sample diluent solution (0.02 M Tris buffer, pH 7.9, 0.5 % BSA, 0.15 M sodium chloride, 0.02% sodium azide).

The negative control was the sample diluent solution used for the positive control without IGFBP-1.

### IGFBP-1 Assay Procedure

Cervicovaginal secretion samples were prepared as described in Example 9. Duplicate 100 µL aliquots of each sample or a dilution thereof, and the positive and negative controls were placed in separate wells of the microtiter plate and incubated for 2 hours at room temperature. Following incubation, the wells were washed three times in rinse buffer (0.02 M Tris, pH 7.9, 0.15 M NaCl, 0.05% TWEEN-20, and 0.02% sodium azide).

Following rinsing, 100 µL of goat anti-IGFBP-1 antibody (1:200 dilution) was added to each well and incubated for 2 hours at room temperature. Following the incubation, the plates were washed three times in rinse buffer. Following rinsing, 100 µL of swine anti-goat conjugate (1:4,000 dilution) was added to each well and incubated for 1 hour at room temperature. Following incubation, the plate was washed once in rinse buffer and 100 µL of enzyme substrate was added to each well. Kinetic absorbance values were read immediately at 405 nm using an ELISA plate reader. The plates were read again after half hour to determine the endpoint reading.

The average absorbance of the duplicate wells for each sample and control was calculated. The IGFBP-1 concentration for the samples was calculated by preparing a standard curve using amniotic fluid with known concentrations of IGFBP-1.

### EXAMPLE 13: Study of a Panel of Subjects

A panel of cervical secretion specimens from second and third trimester subjects was tested for fetal fibronectin as described in Example 10. The panel was tested for rupture of membranes using conventional ferning, pooling, and nitrazine. The same panel was then tested for IGFBP-1.

IGFBP-1 was not detectable (< 10 ng/mL) below to 40 ng/mL in specimens that were negative for rupture of membranes by ferning, pooling, and nitrazine. Furthermore, IGFBP-1 was negative in specimens from women who were fetal fibronectin positive (> 50 ng/mL), rupture of membranes negative (by ferning, pooling, and nitrazine) and either pre-term delivery positive or negative as determined by outcome (whether the subject delivered at or before 37 weeks gestation). Most subjects who were rupture of membranes positive by ferning, pooling, and nitrazine were also positive for IGFBP-1 (range 30 to > 5000 ng/mL).

The circulating levels of IGFBP-1 in maternal plasma were examined to determine if blood contamination of cervicovaginal secretions, interfered with the test for IGFBF-1 . The levels of IGFBP-1 in maternal plasma ranged from less than 10 ng/mL to 250 ng/mL and averaged about 150 ng/mL. Most of the rupture of membranes-positive cervicovaginal secretions specimens registered levels of IGFBP-1 of greater than 250 ng/mL. Thus, levels of IGFBP-1 in cervicovaginal secretions are a reliable indicator of rupture of membranes (ROM) in the presence of 10% blood or absence of blood in cervicovaginal secretion samples. Moreover, when fetal fibronectin is positive (> 50 ng/mL) the absence of IGFBP-1 is a reliable indicator that rupture of membranes has not occurred even though fetal fibronectin is present.

The ability to rule-out rupture of membranes assists the physician in determining the approach to clinical management of the pregnancy.

### EXAMPLE 14: Study of a Panel of Subjects

In a second panel of subjects, four groups of pregnant women were tested for fetal fibronectin and IGFBP-1 in cervical secretions. The groups were:
Group 1: those subjects who were pre-term delivery positive (PTD + ; delivery before 37 weeks) and fetal fibronectin positive (fFN+; > 50 ng/mL) but negative for rupture of membranes (ROM-) by ferning, pooling and nitrazine;
Group 2: those who were PTD- (delivery after 37 weeks) but who exhibited an fFN + test (> 50 ng/mL) and were ROM- by ferning, pooling, and nitrazine;
Group 3: those who were PTD- (delivery after 37 weeks) and fFN-(< 50 ng/mL) and ROM- by ferning, pooling, and nitrazine; and
Group 4: those that were rupture of membranes positive (ROM +) by ferning, pooling, and nitrazine testing regardless of gestational age.

In Group 1, 23 out of 24 cervical secretion specimens exhibited less than 20 ng/mL IGFBP-1, while one specimen from a subject sampled at 27 weeks gestation exhibited 42 ng/mL IGFBP-1 in a specimen that also demonstrated greater than 1 µg of fetal fibronectin per mL.
Therefore, if the cut-off of the rupture of membranes (ROM) rule-out test was 50 ng IGFBP-1 per mL, the rule-out specificity (ROM rule-out specificity is the number of IGFBP-1 negative divided by the number of true ROM negative) would be 100% based upon ROM diagnosis by ferning, pooling, and nitrazine testing. If the cut-off were below 40 ng IGFBP-1 per mL, the ROM rule-out specificity would be 96%.

In Group 2, 27 out of 27 cervical secretion specimens exhibited less than 20 ng/mL IGFBP-1. Thus, in this group, the rule-out of ROM was 100% specific based upon ROM diagnosis by ferning, pooling and nitrazine testing.

In Group 3, 23 out of 23 cervical secretion specimens exhibited less than 20 ng/mL IGFBP-I. Thus, in this group, rule out of ROM was also 100% specific based upon ROM diagnosis by ferning, pooling and nitrazine testing.

In Group 4, 24 out of 30 specimens exhibited greater than 20 ng/mL IGFBP-1 . Thus, in this group of subjects who were diagnosed as ROM + based upon ferning, pooling, and nitrazine testing, the IGFBP-1 test was 80% specific at diagnosing ROM. One of the six IGFBP-1 negative subjects also exhibited a negative fetal fibronectin test (< 50 ng/mL) which should have been positive if amniotic fluid is present since fetal fibronectin is present in amniotic fluid.

The finding that two abundant markers of amniotic fluid (IGFBP-1 and fetal fibronectin) gave results which were in disagreement with the results of the more subjective criteria of ferning, pooling, and nitrazine results calls into question the reliability of ferning, pooling and nitrazine testing for accurate diagnosis of rupture. Ferning, pooling, and nitrazine are well known to be a combination of tests which are inadequate for the determination of rupture. Specifically, when the test result is positive, amniotic fluid is likely to be present.

A negative result cannot indicate that amniotic fluid is absent, since the sensitivity of the test is low. However, since the test is subjective, positive and negative results can be incorrect.

Since modifications will be apparent to those of skill in this art, it is intended that this invention be limited only by the scope of the appended claims.

## Claims

1. 17-alpha-hydroxyprogesterone or 17-alpha-hydroxyprogesterone caproate for use in delaying delivery in a subject at risk for pre-term delivery; wherein the subject has previously been identified as being at risk of preterm delivery by detecting foetal fibronectin at a level indicative of a risk of preterm delivery in a sample isolated from the subject between 20 weeks gestation and 36 weeks gestation wherein the level of indicative of risk is a minimum threshold value of about 50 ng/mL

2. The 17-alpha-hydroxyprogesterone or 17-alpha-hydroxyprogesterone caproate for use in delaying delivery in a subject of claim 1, wherein, wherein the sample contains a body fluid or a swab of the posterior fornix, the cervical canal, the ectocervix and/or the external cervical os.

3. The 17-alpha-hydroxyprogesterone or 17-alpha-hydroxyprogesterone caproate for use in delaying delivery in a subject of claim 1, wherein the administration of the 17-alpha-hydroxyprogesterone or 17-alpha-hydroxyprogesterone caproate is stopped at about 36 weeks of gestation or at the onset of spontaneous labor.

4. The 17-alpha-hydroxyprogesterone or 17-alpha-hydroxyprogesterone caproate for use in delaying delivery in a subject of claim 1, wherein the therapeutically effective amount of the 17-alpha-hydroxyprogesterone or 17-alpha-hydroxyprogesterone caproate comprises at least about 100 mg/week of the 17-alpha-hydroxyprogesterone or 17-alpha-hydroxyprogesterone caproate.

5. The 17-alpha-hydroxyprogesterone or 17-alpha-hydroxyprogesterone caproate for use in delaying delivery in a subject of claim 1, wherein the 17-alpha-hydroxyprogesterone or 17-alpha-hydroxyprogesterone caproate is administered orally, by intramuscular injection, transdermally, or intranasally.

6. The 17-alpha-hydrotyprogesterone or 17-alpha-hydroxyprogesterone caproate for use in delaying delivery in a subject of claim 1, wherein the level of foetal fibronectin is determined by the steps of: a) contacting the sample with an anti-(foetal fibronectin) antibody for a time sufficient to permit antigen-antibody binding to occur; b) contacting the sample with an insoluble support, to which anti-fibronectin antibody is adhered, for a time sufficient to permit antigen-antibody binding to occur; and c) detecting anti-(foetal fibronectin) antibody on the insoluble support.

7. The 17-alpha-hydroxyprogesterone or 17-alpha-hydroxyprogesterone caproate for use in delaying delivery in a subject of claim 6, wherein material from the sample is contacted with the insoluble support in a region of the insoluble support that contains mobilizable anti-(foetal fibronectin) antibody.

8. The 17-alpha-hydroxyprogesterone or 17-alpha-hydroxyprogesterone caproate for use in delaying delivery in a subject of claim6, wherein the anti-(foetal fibronectin) antibody is conjugated to a physically detectable label.

9. The 17-alpha-hydroxyprogesterone or 17-alpha-hydroxyprogesterone caproate for use in delaying delivery in a subject of claim 6, wherein the step of detecting anti-(foetal fibronectin) antibody comprises the steps of: a) contacting the insoluble support with a labelled antibody which binds selectively with the anti-(foetal fibronectin) antibody; and b) detecting the label on the insoluble support.

10. The 17-alpha-hydroxyprogesterone or 17-alpha-hydroxyprogesterone caproate for use in delaying delivery in a subject of claim 1, wherein the level of foetal fibronectin is determined by the steps of: a) contacting the sample with an anti-fibronectin antibody for a time sufficient to permit antigen-antibody binding to occur; and b) detecting formation of an antibody-antigen complex.

11. The 17-alpha-hydroxyprogesterone or 17-alpha-hydroxyprogesterone caproate for use in delaying delivery in a subject of claim 10, wherein the step of detecting formation of an antibody-antigen complex further comprises the steps of: c) contacting the sample with an insoluble support comprising an immobilized an anti-(foetal fibronectin) antibody under conditions, whereby foetal fibronectin in the sample binds to the antibody; and d) detecting the anti-fibronectin antibody on the insoluble support.

12. The 17-alpha-hydrozyprogesterone or 17-alpha-hydrotyprogesterone caproate for use in delaying delivery in a subject of claim 10, wherein the anti-tibronectin antibody comprises a detectable label.

13. The 17-alpha-hydroxyprogesterone or 17-alpha-hydroxyprogesterone caproate for use in delaying delivery in a subject of claim 10, wherein the step of detecting the anti-fibronectin antibody comprises the steps of: e) contacting the insoluble support with a labeled antibody that binds selectively with the anti-fibronectin antibody; and f) detecting the label on the insoluble support.

## Patentansprüche

1. 17-alpha-Hydroprogesteron oder 17-alpha-Hydroprogesteron-Caproat zur Verwendung zur Verzögerung der Entbindung in einem Individuum, welches für eine Frühgeburt gefährdet ist; wobei das Individuum vorher als gefährdet für eine Frühgeburt identifiziert worden ist, durch den Nachweis von fötalem Fibronektin mit einem Level, welches auf eine Gefahr für eine Frühgeburt hinweist, in einer Probe, welche aus dem Individuum zwischen der 20. Schwangerschaftswoche und der 36. Schwangerschaftswoche isoliert worden ist, wobei das Level, welches auf eine Gefahr hinweist, einen Minimum-Schwellenwert von ungefähr 50 ng/mL hat.

2. 17-alpha-Hydroprogesteron oder 17-alpha-Hydroprogesteron-Caproat zur Verwendung zur Verzögerung der Entbindung in einem Individuum nach Anspruch 1, wobei die Probe eine Körperflüssigkeit oder einen Abstrich des hinteren Scheidengewölbes, des Gebärmutterhalskanals, der Ektozervix und/oder des externen Muttermundes enthält.

3. 17-alpha-Hydroprogesteron oder 17-alpha-Hydroprogesteron-Caproat zur Verwendung zur Verzögerung der Entbindung in einem Individuum nach Anspruch 1, wobei die Verabreichung des 17-alpha-Hydroprogesterons oder des 17-alpha-Hydroprogesteron-Caproats in ungefähr der 36. Schwangerschaftswoche oder beim Einsetzen spontaner Wehen gestoppt wird.

4. 17-alpha-Hydroprogesteron oder 17-alpha-Hydroprogesteron-Caproat zur Verwendung zur Verzögerung der Entbindung in einem Individuum nach Anspruch 1, wobei die therapeutisch effektive Menge des 17-alpha-Hydroprogesterons oder des 17-alpha-Hydroprogesteron-Caproats mindestens ungefähr 100 mg/Woche des 17-alpha-Hydroprogesterons oder des 17-alpha-Hydroprogesteron-Caproats umfasst.

5. 17-alpha-Hydroprogesteron oder 17-alpha-Hydroprogesteron-Caproat zur Verwendung zur Verzögerung der Entbindung in einem Individuum nach Anspruch 1, wobei das 17-alpha-Hydroprogesteron oder das 17-alpha-Hydroprogesteron-Caproat oral, durch intramuskuläre Injektion, transdermal oder intranasal verabreicht wird.

6. 17-alpha-Hydroprogesteron oder 17-alpha-Hydroprogesteron-Caproat zur Verwendung zur Verzögerung der Entbindung in einem Individuum nach Anspruch 1, wobei das Level an fötalem Fibronektin bestimmt wird durch die Schritte: a) in Kontakt bringen der Probe mit einem anti-(fötales Fibronektin) Antikörper für einen Zeitraum, der ausreicht um zuzulassen, dass Antigen-Antikörper-Bindung stattfindet; b) in Kontakt bringen der Probe mit einem unlöslichen Träger, an welchen der anti-Fibronektin-Antikörper haftet für einen Zeitraum der ausreicht um zuzulassen, dass Antigen-Antikörper-Bindung stattfindet; und c) Nachweisen von anti-(fötales Fibronektin) Antikörper auf dem unlöslichen Träger.

7. 17-alpha-Hydroprogesteron oder 17-alpha-Hydroprogesteron-Caproat zur Verwendung zur Verzögerung der Entbindung in einem Individuum nach Anspruch 6, wobei Material der Probe mit dem unlöslichen Träger in einem Bereich des Trägers in Kontakt gebracht wird, die mobilisierbaren anti-(fötales Fibronektin) Antikörper enthält.

8. 17-alpha-Hydroprogesteron oder 17-alpha-Hydroprogesteron-Caproat zur Verwendung zur Verzögerung der Entbindung in einem Individuum nach Anspruch 6, wobei der anti-(fötales Fibronektin) Antikörper an einen physikalisch nachweisbaren Marker konjugiert ist.

9. 17-alpha-Hydroprogesteron oder 17-alpha-Hydroprogasteron-Caproat zur Verwendung zur Verzögerung der Entbindung in einem Individuum nach Anspruch 6, wobei der Schritt des Nachweisens von anti-(fötales Fibronektin) Antikörper die Schritte umfasst: a) in Kontakt bringen des unlöslichen Trägers mit einem markierten Antikörper, welcher selektiv mit dem anti-(fötales Fibronektin) Antikörper bindet; und b) Nachweisen des Markers auf dem unlöslichen Träger.

10. 17-alpha-Hydroprogesteron oder 17-alpha-Hydroprogesteron-Caproat zur Verwendung zur Verzögerung der Entbindung in einem Individuum nach Anspruch 1, wobei das Level an fötalem Fibronektin bestimmt wird durch die Schritte: a) in Kontakt bringen der Probe mit einem anti-Fibronektin Antikörper für einen Zeitraum, der ausreicht um zuzulassen, dass Antigen-Antikörper-Bindung stattfindet; und b) Nachweisen der Bildung eines Antikörper-Antigen-Komplexes.

11. 17-alpha-Hydroprogesteron oder 17-alpha-Hydroprogesteron-Caproat zur Verwendung zur Verzögerung der Entbindung in einem Individuum nach Anspruch 10, wobei der Schritt des Nachweisens der Bildung des Antikörper-Antigen-Komplexes zusätzlich die Schritte umfasst: c) in Kontakt bringen der Probe mit einem unlöslichen Träger, der einen immobilisierten anti-(fötales Fibronektin) Antikörper umfasst, unter Bedingungen, wobei fötales Fibronektin in der Probe an den Antikörper bindet; und d) Nachweisen des anti-Fibronektin Antikörpers auf dem unlöslichen Träger.

12. 17-alpha-Hydroprogesteron oder 17-aipha-Hydroprogosteron-Caproat zur Verwendung zur Verzögerung der Entbindung in einem Individuum nach Anspruch 10, wobei der anti-Fibronektin Antikörper einen nachweisbaren Marker umfasst.

13. 17-alpha-Hydroprogesteron oder 17-alpha-Hydroprogesteron-Caproat zur Verwendung zur Verzögerung der Entbindung in einem Individuum nach Anspruch 10, wobei der Schritt des Nachweisens des anti-Fibronektin Antikörpers die Schritte umfasst: e) in Kontakt bringen des unlöslichen Trägers mit einem markierten Antikörper, der selektiv an den anti-Fibronektin Antikörper bindet; und f) Nachweisen des Markers auf dem unlöslichen Träger.

## Revendications

1. 17-alpha-hydroxyprogestérone ou caproate de 17-alpha-hydroxyprogestérone pour une utilisation afin de retarder l'accouchement chez un sujet présentant un risque d'accouchement avant terme ; où le sujet a été préalablement identifié en tant que sujet à risque d'accouchement avant terme en détectant la fibronectine foetale à un taux indiquant un risque d'accouchement avant terme dans un échantillon isolé du sujet entre 20 semaines de gestation et 36 semaines de gestation, où le taux d'indication de risque est une valeur seuil minimale d'environ 50 ng/mL.

2. 17-alpha-hydroxyprogestérone ou caproate de 17-alpha-hydroxyprogestérone pour une utilisation afin de retarder l'accouchement chez un sujet suivant la revendication 1, où l'échantillon contient un fluide corporel ou un échantillon d'écouvillonnage du cul de sac postérieur du vagin, du canal du col de l'utérus, de l'ectocervix et/ou de l'orifice externe du col de l'utérus.

3. 17-alpha-hydroxyprogestérone ou caproate de 17-alpha-hydroxyprogestérone pour une utilisation afin de retarder l'accouchement chez un sujet suivant la revendication 1, où l'administration de la 17-alpha-hydroxyprogestérone ou du caproate de 17-alpha-hydroxyprogestérone est interrompue à environ 36 semaines de gestation ou bien au début du travail spontané.

4. 17-alpha-hydroxyprogestérone ou caproate de 17-alpha-hydroxyprogestérone pour une utilisation afin de retarder l'accouchement chez un sujet suivant la revendication 1, où la quantité thérapeutiquement efficace de la 17-alpha-hydroxyprogestérone ou du caproate de 17-alpha-hydroxyprogestérone comprend au moins environ 100 mg/semaine de la 17-alpha-hydroxyprogestérone ou du caproate de 17-alpha-hydroxyprogestérone.

5. 17-alpha-hydroxyprogestérone ou caproate de 17-alpha-hydroxyprogestérone pour une utilisation afin de retarder l'accouchement chez un sujet suivant la revendication 1, où la 17-alpha-hydroxyprogestérone ou le caproate de 17-alpha-hydroxyprogestérone est administré par voie orale, par injection intramusculaire, par voie transdermique ou par voie intranasale.

6. 17-alpha-hydroxyprogestérone ou caproate de 17-alpha-hydroxyprogestérone pour une utilisation afin de retarder l'accouchement chez un sujet suivant la revendication 1, où le taux de fibronectine foetale est déterminé par les étapes consistant : a) à mettre en contact l'échantillon avec un anticorps anti-(fibronectine foetale) pendant une période suffisante pour permettre à la liaison antigène/anticorps de se produire ; b) à mettre en contact l'échantillon avec un support insoluble, auquel adhère un anticorps anti-fibronectine, pendant une période de temps suffisante pour permettre à la liaison antigène-anticorps de se produire ; et c) à détecter l'anticorps anti-(fibronectine foetale) sur le support insoluble.

7. 17-alpha-hydroxyprogestérone ou caproate de 17-alpha-hydroxyprogestérone pour une utilisation afin de retarder l'accouchement chez un sujet suivant la revendication 6, où la substance de l'échantillon est mise en contact avec le support insoluble dans une région du support insoluble qui contient un anticorps anti-(fibronectine foetale) mobilisable.

8. 17-alpha-hydroxyprogestérone ou caproate de 17-alpha-hydroxyprogestérone pour une utilisation afin de retarder l'accouchement chez un sujet suivant la revendication 6, où l'anticorps anti-(fibronectine foetale) est conjugué avec un marqueur détectable physiquement.

9. 17-alpha-hydroxyprogestérone ou caproate de 17-alpha-hydroxyprogestérone pour une utilisation afin de retarder l'accouchement chez un sujet suivant la revendication 6, où l'étape de détection de l'anticorps anti-(fibronectine foetale) comprend les étapes consistant : a) à mettre en contact le support insoluble avec un anticorps marqué qui se lie sélectivement à l'anticorps anti-(fibronectine foetale) ; b) à détecter le marqueur sur le support insoluble.

10. 17-alpha-hydroxyprogestérone ou caproate de 17-alpha-hydroxyprogestérone pour une utilisation afin de retarder l'accouchement chez un sujet suivant la revendication 1, où le taux de fibronectine foetale est déterminé par les étapes consistant : a) à mettre en contact l'échantillon avec un anticorps anti-fibronectine pendant une période de temps suffisante pour permettre à la liaison antigène-anticorps de se produire ; et b) à détecter la formation d'un complexe anticorps-antigène.

11. 17-alpha-hydroxyprogestérone ou caproate de 17-alpha-hydroxyprogestérone pour une utilisation afin de retarder l'accouchement chez un sujet suivant la revendication 10, où l'étape de détection de la formation d'un complexe anticorps-antigène comprend en outre les étapes consistant : c) à mettre en contact l'échantillon avec un support insoluble comprenant un anticorps anti-(fibronectine foetale) immobilisé dans des conditions permettant la liaison de la fibronectine foetale présente dans l'échantillon au support ; et d) à détecter l'anticorps anti-fibronectine sur le support insoluble.

12. 17-alpha-hydroxyprogestérone ou caproate de 17-alpha-hydroxyprogestérone pour une utilisation afin de retarder l'accouchement chez un sujet suivant la revendication 10, où l'anticorps anti-fibronectine comprend un marqueur détectable.

13. 17-alpha-hydroxyprogestérone ou caproate de 17-alpha-hydroxyprogestérone pour une utilisation afin de retarder l'accouchement chez un sujet suivant la revendication 10, où l'étape de détection de l'anticorps anti-fibronectine comprend les étapes consistant : e) à mettre en contact le support insoluble avec un anticorps marqué qui se lie sélectivement à l'anticorps anti-fibronectine ; et f) à détecter le marqueur sur le support insoluble.
